# EUROPEAN PATENT APPLICATION

(11) **EP 1 484 322 A1**
(43) Date of publication of application: **08.12.2004**
(21) Application number: 03708541.2
(22) Date of filing: 11.03.2003
(51) Int. Cl.: C07D 233/64, C07D 403/12, A61K 31/4164, A61K 31/55, A61K 31/4178, A61P 43/00

(54) **PROCESS FOR PRODUCING OPTICALLY ACTIVE SULFOXIDE DERIVATIVE**

(30) Priority: 12.03.2002 JP 2002066809; 07.08.2002 JP 2002229802
(71) Applicant: Takeda Pharmaceutical Company Limited, Osaka 540-08645 (JP)
(72) Inventor: TAWADA, Hiroyuki, Takatsuki-shi, Osaka 569-1032 (JP); IKEMOTO, Tomomi, Takarazuka-shi, Hyogo 665-0815 (JP); NISHIGUCHI, Atsuko, Itami-shi, Hyogo 664-0883 (JP); ITO, Tatsuya, 117.1-7 Asahigaoka-Kukakuseirichinai, Kashiba-shi, Nara 639-0264 (JP); ADACHI, Mari, Kobe-shi, Hyogo 651-2216 (JP)
(74) Representative: von Kreisler, Alek, Dipl.-Chem.
(86) International application number: PCT/JP2003/002840
(87) International publication number: WO 2003/076411

(57) **Abstract**

A process for preparing an optically active sulfoxide derivative (I) having CCR5 antagonism without causing side reactions such as racemization and Pummerer rearrangement, which comprises reacting a compound (II) with a compound (III) as shown by the following scheme: 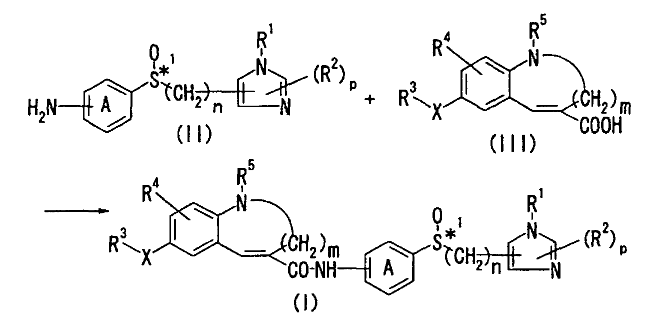 wherein R¹ represents hydrogen, an aliphatic hydrocarbon group or an aromatic group; R² represents halogeno, alkyl, hydroxyl, amino, an aromatic group, etc.; R³ represents a 5- or 6-membered ring; R⁴ represents hydrogen, alkyl, alkoxy or halogeno; R⁵ represents hydrogen, a hydrocarbon group, a heterocyclic group, acyl, etc.; ring A represents an optionally substituted benzene ring; X represents a bond or divalent group comprising a linear part constituted of 1 to 4 atoms; m represents an integer of 1 to 5; n represents an integer of 0 to 3; p represents an integer of 0 to 2; and ^{*1} represents an asymmetric center.

## Description

### Technical Field

The present invention relates to an industrially advantageous process for preparing an optically active sulfoxide derivative having CCR5 antagonism, or a salt thereof.

### Background Art

### Prior Art

Conventionally, as a process for preparing an optically active sulfoxide derivative, separation by a chiral column is generally employed, except for special cases. However, such a process is not a fully satisfactory one from an industrial point of view, because it requires a special apparatus such as SMB (Simulated moving bed).

### Disclosure of Prior Art

The prior art documents related to the present invention are as follows:
[Patent Document 1]
   WO96/01267 A
[Patent Document 2]
   WO99/32468 A
[Patent Document 3]
   WO99/3210 A
[Patent Document 4]
   WO00/10965 A
[Patent Document 5]
   WO00/37455 A
[Patent Document 6]
   WO00/68203 A
[Patent Document 7]
   WO00/76993 A
[Patent Document 8]
   GB Patent No. 1579270
[Patent Document 9]
   JP 62-265270 A
[Patent Document 10]
   JP 2002-521408 A
[Patent Document 11]
   WO01/46203 A

### [Non-Patent Document 1]

Proc. Natl. Acad. Sci. (USA), Volume 96, pp. 5698-5703 (May, 1999)

### Problems to be Solved by the Invention

The present invention provides an industrially advantageous process for preparing an optically active sulfoxide derivative or a salt thereof, by acylating an optically active sulfoxide derivative having an amino group in the molecule without causing side reactions such as racemization and Pummerer rearrangement.

### Brief Disclosure of the Invention

The present inventors have studied a process for preparing an optically active sulfoxide derivative having CCR5 antagonism or a salt thereof, intensively, and as a result, they have found an industrially advantageous process for preparing an optically active sulfoxide derivative or a salt thereof without causing side reactions such as racemization and Pummerer rearrangement.

Namely, the present invention relates to:
(1) A process for preparing an optically active compound represented by the formula (I): 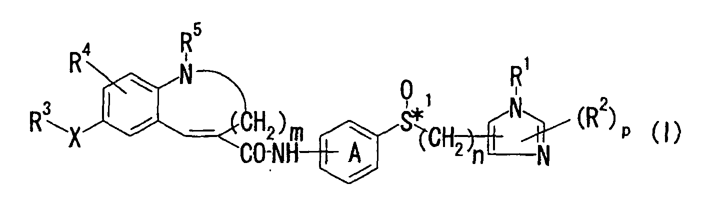 wherein R¹ represents a hydrogen atom, an optionally substituted aliphatic hydrocarbon group or an optionally substituted aromatic group;
   R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
   R³ represents an optionally substituted 5- or 6-membered ring;
   R⁴ represents a hydrogen atom, an optionally substituted lower alkyl group, an optionally substituted lower alkoxy group or a halogen atom;
   R⁵ represents a hydrogen atom, an optionally substituted hydrocarbon group, an optionally substituted heterocyclic group, an optionally substituted sulfonyl group, an esterified or amidated carboxyl group or an optionally substituted acyl group;
   X represents a bond or a divalent group comprising a linear part constituted of 1 to 4 atoms;
   the ring A represents a benzene ring which may be substituted with a halogen atom, a C₁₋₄ alkyl group which may be substituted with a halogen atom or a C₁₋₄ alkoxy group which may be substituted with a halogen atom;
   m is an integer of 1 to 5;
   n represents an integer of 0 to 3;
   p represents an integer of 0 to 2;
   ^{*1} represents an asymmetric center; and
   the SO to which ^{*1} is given represents an asymmetric sulfoxide group,
   or a salt thereof, which comprises reacting an optically active compound represented by the formula (II): 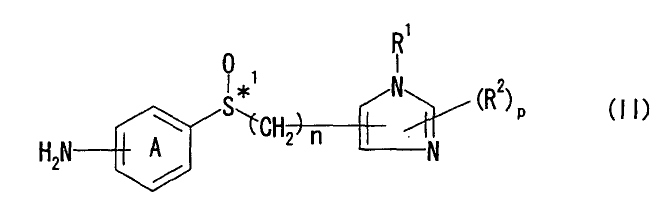 wherein each symbol is as defined above, or a salt thereof, with a compound represented by the formula (III): 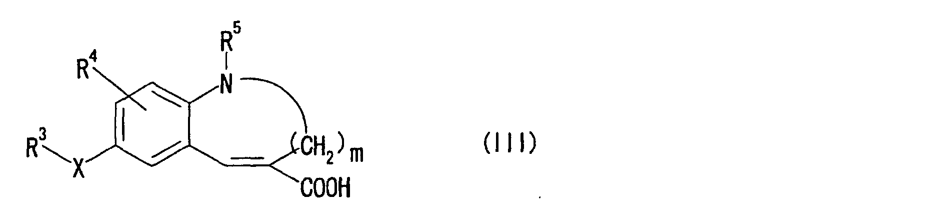 wherein each symbol is as defined above, a salt thereof or a reactive derivative thereof;
(2) A process for preparing an optically active compound represented by the formula (I) or a salt thereof, which comprises reacting an optically active compound represented by the formula (XIa): 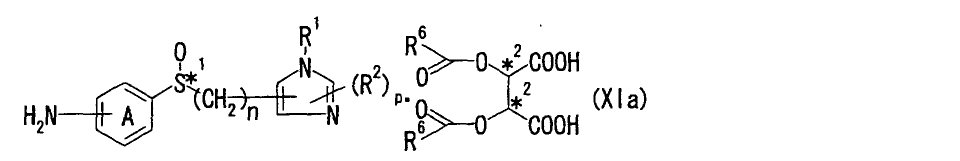 wherein R⁶ represents a methyl group, a phenyl group, a 4-methylphenyl group or a α-naphthyl group;
   ^{*2} represents an asymmetric center; and
   the other symbols are as defined above,
   or an optically active compound represented by the formula (XIb): 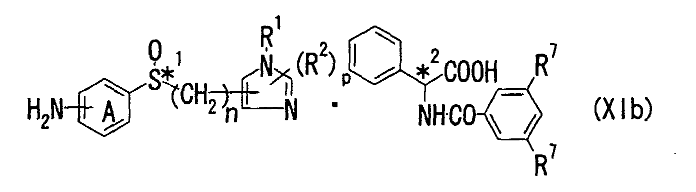 wherein R⁷ represents a hydrogen atom, a chlorine atom or a nitro group; and
   the other symbols are as defined above,
   with a compound represented by the formula (III), a salt thereof or a reactive derivative thereof;
(3) An optically active compound represented by the formula (II) or a salt thereof;
(4) The optically active compound as described in the above (3), wherein R¹ and R² each represents a C₁₋₆ alkyl group, and n represents 1 or 2, or a salt thereof;
(5) The optically active compound as described in the above (3), wherein R¹ represents a C₁₋₆ alkyl group, p represents 0; n represents 1, and 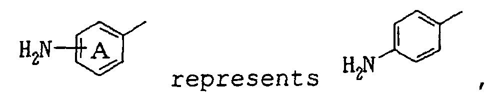 or a salt thereof;
(6) A process for preparing an optically active compound represented by the formula (II) or a salt thereof, which comprises subjecting an optically active compound represented by the formula (XIa) or the formula (XIb) to a metathesis reaction;
(7) An optically active compound represented by the formula (XIa) or the formula (XIb);
(8) The optically active compound as described in the above (7), wherein R¹ and R² each represents a C₁₋₆ alkyl group, n represents 1 or 2, and R⁶ represents a 4-methylphenyl group or R⁷ represents a nitro group;
(9) The optically active compound as described in the above (7), wherein R¹ represents a C₁₋₆ alkyl group, p represents 0; n represents 1 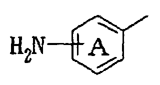 represents 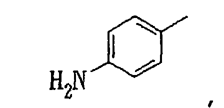 and R⁶ represents a 4-methylphenyl group or R⁷ represents a nitro group;
(10) A process for preparing an optically active compound represented by the formula (XIa) or the formula (XIb), which comprises subjecting a compound represented by the formula (IX): 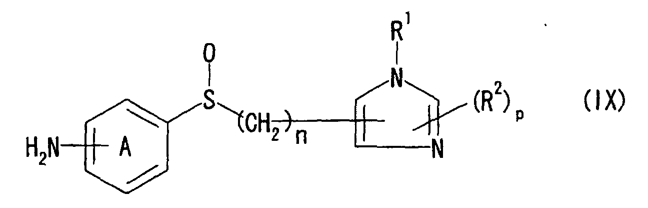 wherein each symbol is as defined above,
   or a salt thereof, to optical resolution with an optically active acid represented by the formula (XIIa): 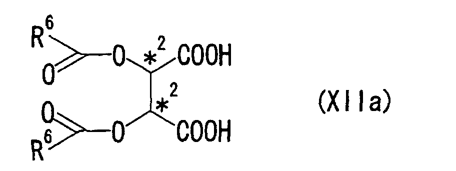 wherein each symbol is as defined above,
   or the formula (XIIb): 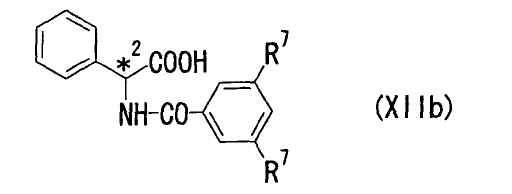 wherein each symbol is as defined above;
(11) A process for preparing an optically active compound represented by the formula (II) or a salt thereof, which comprises oxidizing a compound represented by the formula (X): 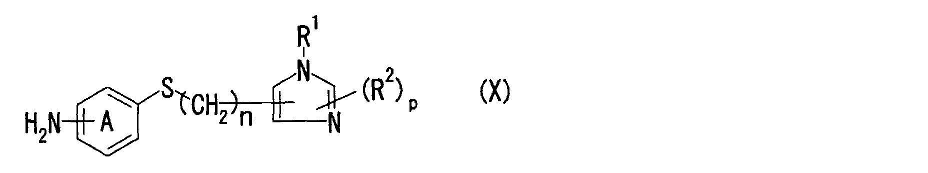 wherein each symbol is as defined above,
   or a salt thereof, in the presence of the formula (XIIa), or an acid which is optically active with respect to axial asymmetry and represented by the formula (XIIc): 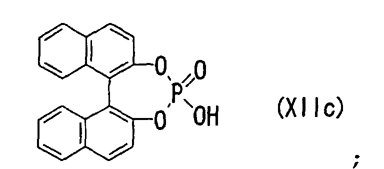
(12) A compound represented by the formula (IX) or a salt thereof;
(13) A process for preparing a compound represented by the formula (IX) or a salt thereof, which comprises subjecting a compound represented by the formula (VIII): 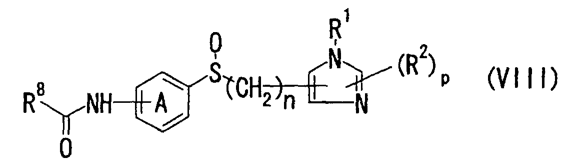 wherein R⁸ represents a hydrogen atom, an optionally substituted lower alkyl group, an optionally substituted aryl group, an optionally substituted aralkyl group or -OR¹⁰ wherein R¹⁰ represents an optionally substituted lower alkyl group, an optionally substituted aryl group or an optionally substituted aralkyl group, and
   the other symbols are as defined above, or a salt thereof, to a deprotection reaction;
(14) A process for preparing a compound represented by the formula (IX) or a salt thereof; which comprises oxidizing a compound represented by the formula (X) or a salt thereof;
(15) A compound represented by the formula (VIII) or a salt thereof;
(16) A process for preparing a compound represented by the formula (VIII) or a salt thereof, which comprises oxidizing a compound represented by the formula (VII): 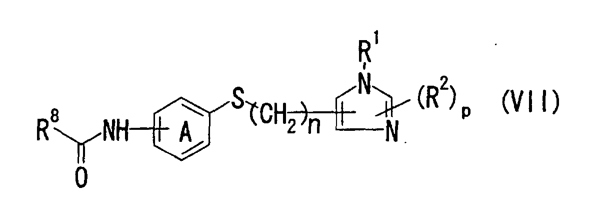 wherein each symbol is as defined above, or a salt thereof;
(17) A compound represented by the formula (VII'): 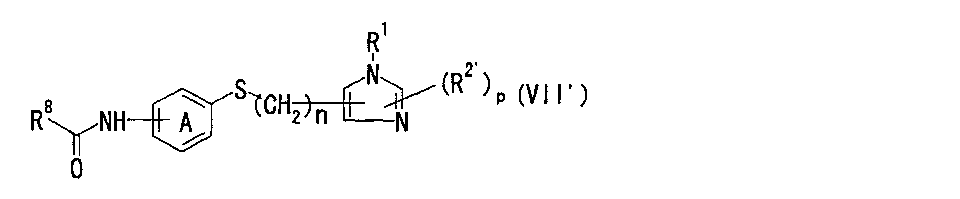 wherein R^{2'} represents a halogen atom, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group, and
   the other symbols are as defined above,
   or a salt thereof;
(18) A process for preparing a compound represented by the formula (VII) or a salt thereof, which comprises reacting a compound represented by the formula (V): 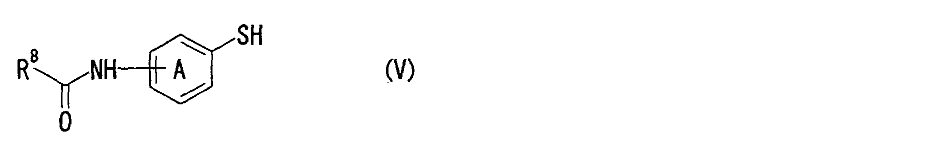 wherein each symbol is as defined above, or a salt thereof, with a compound represented by the formula (VI): 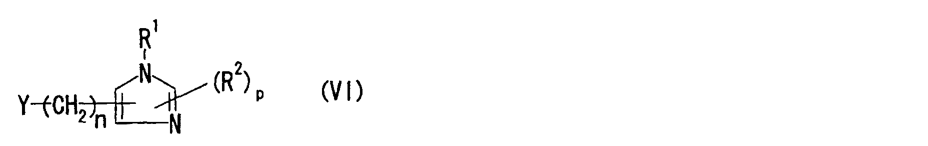 wherein Y represents a halogen atom or a group represented by the formula -OSO₂-R⁹ wherein R⁹ represents a lower alkyl group or an optionally substituted aryl group, and
   the other symbols are as defined above, or a salt thereof;
(19) A compound represented by the formula (X'): 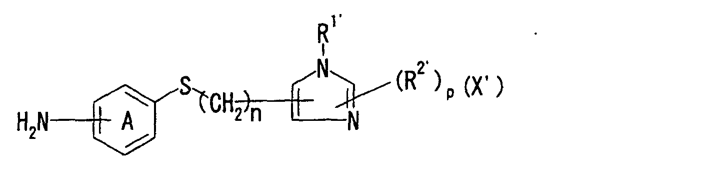 wherein each symbol is as defined above,
   or a salt thereof; and
(20) A process for preparing a compound represented by the formula (X) or a salt thereof, which comprises reacting a compound represented by the formula (IV): 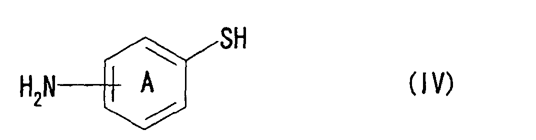 wherein the ring A is as defined above,
   or a salt thereof, with a compound represented by the formula (VI), or a salt thereof.

### Detailed Description of the Invention

Hereinafter, the present invention will be explained in detail.

A halogen atom which is the substituent of the ring A includes, for example, fluorine, chloride and bromine; a C₁₋₄ alkyl group which may be substituted with a halogen atom includes, for example, methyl, ethyl, trifluoromethyl and trifluoroethyl; and a C₁₋₄ alkoxy group which may be substituted with a halogen atom includes, for example, methoxy, ethoxy, propoxy, trifluoromethoxy and trifluoroethoxy.

An optionally substituted aliphatic hydrocarbon group represented by R¹ includes an optionally substituted alkyl group, an optionally substituted alkenyl group, an optionally substituted alkynyl group, an optionally substituted cycloalkyl group and an optionally substituted cycloalkenyl group.

The alkyl group in the optionally substituted alkyl group includes a straight-chain or branched C₁₋₁₀ alkyl group, for example, a C₁₋₁₀ alkyl group such as methyl, ethyl, propyl, isopropyl, butyl, isobutyl, sec-butyl, tert-butyl, pentyl, isopentyl, neopentyl, hexyl, heptyl, octyl, nonyl and decyl, preferably a lower C₁₋₆ alkyl group; the alkenyl group in the optionally substituted alkenyl group includes, for example, a straight-chain or branched C₂₋₁₀ alkenyl group having 1 to 5 double bonds, for example, a C₁₋₁₀ alkenyl group such as ethenyl, propenyl, butenyl, isobutenyl, pentenyl, hexenyl, heptenyl, octenyl, noneyl and decenyl, preferably a lower C₁₋₆ alkenyl group; the alkynyl group in the optionally substituted alkynyl group includes, for example, a straight-chain or branched C₂₋₁₀ alkynyl group having 1 to 5 double bonds, for example, a C₁₋₁₀ alkynyl group such as ethynyl, propynyl, butynyl, isobutynyl, pentynyl, hexynyl, heptynyl, octynyl, nonynyl and decynyl, preferably a lower C₁₋₆ alkynyl group, repectively. The substituent in the optionally substituted alkyl group, the optionally substituted alkenyl group or the optionally substituted alkynyl group, includes a halogen atom (e.g., fluorine, chlorine, bromine, iodine, etc.), a nitro group, a cyano group, an optionally substituted hydroxyl group (e.g., a hydroxyl group, C₁₋₄ alkoxy, etc.), an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5- or 6-membered cyclic amino group such as, pyrrolidine, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

The cycloalkyl group in the optionally substituted cycloalkyl group includes, for example, a C₃₋₇ cycloalkyl group, etc. such as cyclopropyl, cyclobutyl, cyclopentyl, cyclohexyl and cycloheptyl, and the cycloalkenyl group in the optionally substituted cycloalkenyl group includes, for example, a C₃₋₇ cycloalkenyl group, etc. such as cyclopropenyl, cyclobutenyl, cyclopentenyl, cyclohexenyl and cycloheptenyl. The substituent in the optionally substituted cycloalkyl group and the optionally substituted cycloalkynyl group includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5- or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), formyl, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

The aromatic group in the optionally substituted aromatic group represented by R¹ includes a 5- or 6-membered homo- or heterocyclic aromatic group such as phenyl, pyridyl, furyl, thienyl, pyrrolyl, imidazolyl, pyrazolyl, thiazolyl, oxazolyl, isothiazolyl, isoxazolyl, tetrazolyl, pyrazinyl, pyrimidinyl, pyridazinyl and triazolyl; and a condensed heterocyclic aromatic group such as benzofuran, indole, benzothiophene, benzoxazole, benzothiazole, indazole, benzimidazole, quinoline, isoquinoline, quinoxaline, phthalazine, quinazoline, cinnoline and imidazopyridine. The substituent in the aromatic group includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5- or 6- membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkyl group (e.g., trifluoromethyl, methyl, ethyl, etc.), an optionally halogenated C₁₋₄ alkoxy group (e.g., methoxy, ethoxy, propoxy, butoxy, trifluoromethoxy, trifluoroethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

The "substituent" which may be contained in the "aromatic group" of the "optionally substituted aromatic group" represented by R¹ preferably includes, in particular, a halogen atom, an optionally halogenated or lower-(C₁₋₄) alkoxylated lower (C₁₋₄) alkyl group (e.g., methyl, ethyl, t-butyl, trifluoromethyl, methoxymethyl, ethoxymethyl, propoxymethyl, butoxymethyl, methoxyethyl, ethoxyethyl, propoxyethyl, butoxyethyl, etc.), a lower (C₁₋₄) alkyl group which may be substituted with a hydroxyl group or a cyano group (e.g., hydroxy C₁₋₄ alkyl, cyano C₁₋₄ alkyl, etc.), a lower (C₁₋₄) alkyl group which may be substituted with an optionally esterified or amidated carboxyl group (e.g., carboxyl C₁₋₄ alkyl, C₁₋₄ alkoxycarbonyl C₁₋₄ alkyl, carbamoyl C₁₋₄ alkyl, mono-C₁₋₄ alkylcarbamoyl C₁₋₄ alkyl, di-C₁₋₄ alkylcarbamoyl C₁₋₄ alkyl, pyrrolidinocarbonyl C₁₋₄ alkyl, piperidinocarbonyl C₁₋₄ alkyl, morpholinocarbonyl C₁₋₄ alkyl, thiomorpholinocarbonyl C₁₋₄ alkyl, etc.), an optionally halogenated or lower-(C₁₋₄) alkoxylated lower (C₁₋₄) alkoxy group (e.g., methoxy, ethoxy, propoxy, butoxy, t-butoxy, trifluoromethoxy, methoxymethoxy, ethoxymethoxy, propoxymethoxy, butoxymethoxy, methoxyethoxy, ethoxyethoxy, propoxyethoxy, butoxyethoxy, methoxypropoxy, ethoxypropoxy, propoxypropoxy, butoxypropoxy, etc.), a halogen atom (e.g., fluorine, chlorine, etc.), a nitro group, a cyano group and 1 or 2 of a lower (C₁₋₄) alkyl group, and more preferably an optionally halogenated lower (C₁₋₄) alkyl group.

The "optionally substituted alkyl group", the "optionally substituted cycloalkyl group" and the "optionally substituted aromatic group" represented by R² are as defined in R¹.

The substituent in the optionally substituted hydroxyl group represented by R² includes (1) an optionally substituted alkyl group (e.g., a C₁₋₁₀ alkyl group such as methyl, ethyl, propyl, isopropyl, butyl, isobutyl, sec-butyl, tert-butyl, pentyl, isopentyl, neopentyl, hexyl, heptyl, octyl, nonyl and decyl, preferably a lower (C₁₋₆) alkyl group, or the like is exemplified); (2) a cycloalkyl group that may be substituted and may contain heteroatoms (e.g., C₃₋₇ cycloalkyl such as cyclopropyl, cyclobutyl, cyclopentyl, cyclohexyl and cycloheptyl; a saturated 5- to 6-membered heterocyclic ring group containing 1 to 2 heteroatoms such as tetrahydrofuranyl, tetrahydrothienyl, pyrrolidinyl, pyrazolidinyl, piperidyl, piperazinyl, morpholinyl, thiomorpholinyl, tetrahydropyranyl and tetrahydrothiopyranyl (preferably, tetrahydropyranyl, etc.); or the like is exemplified); (3) an optionally substituted alkenyl group (e.g., a C₂₋₁₀ alkenyl group such as allyl, crotyl, 2-pentenyl and 3-hexenyl, preferably a lower (C₂₋₆) alkenyl group, or the like is exemplified); (4) an optionally substituted cycloalkenyl group (e.g., a C₃₋₇ cycloalkenyl group such as 2-cyclopentenyl, 2-cyclohexenyl, 2-cyclopentenylmethyl and 2-cyclohexenylmethyl, or the like is exemplified); (5) an optionally substituted aralkyl group (e.g., phenyl-C₁₋₄ alkyl (e.g., benzyl, phenethyl, etc.), or the like is exemplified); (6) a formyl group or an optionally substituted acyl group (e.g., a C₂₋₄ alkanoyl group such as acetyl, propionyl, butyryl and isobutyryl, and a C₁₋₄ alkylsulfonyl group such as methanesulfonyl and ethanesulfonyl, or the like is exemplified); and (7) an optionally substituted aryl group (e.g., phenyl, naphthyl, or the like is exemplified). The substituent which may be contained in (1) an optionally substituted alkyl group, (2) an optionally substituted cycloalkyl group, (3) an optionally substituted alkenyl group, (4) an optionally substituted cycloalkenyl group, (5) an optionally substituted aralkyl group, (6) an optionally substituted acyl group, and (7) an optionally substituted aryl group, includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5-or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkyl group (e.g., trifluoromethyl, methyl, ethyl, etc.), an optionally halogenated C₁₋₆ alkoxy group (e.g., methoxy, ethoxy, propoxy, butoxy, trifluoromethoxy, trifluoroethoxy, etc.; preferably optionally halogenated C₁₋₄ alkoxy), a formyl group, a C₂₋₄ alkanoyl group (e.g., acetyl, propionyl, etc.), a C₁₋₄ alkylsulfonyl group (e.g., methanesulfonyl, ethanesulfonyl, etc.), a 5- to 6-membered aromatic heterocycle that may be substituted [e.g., a 5- to 6-membered aromatic heterocycle, which contains 1 to 4 heteroatoms of 1 to 2 kinds selected from a nitrogen atom, a sulfur atom and an oxygen atom, such as furan, thiophene, pyrrole, imidazole, pyrazole, thiazole, oxazole, isothiazole, isoxazole, tetrazole, pyridine, pyrazine, pyrimidine, pyridazine and triazole; the substituent that may be contained in the heterocycle, includes a halogen atom (e.g., fluorine, chlorine, bromine, iodine, etc.), a nitro group, a cyano group, a hydroxyl group, a thiol group, an amino group, a carboxyl group, an optionally halogenated C₁₋₄ alkyl group (e.g., trifluoromethyl, methyl, ethyl, etc.), an optionally substituted C₁₋₄ alkoxy group (e.g., methoxy, ethoxy, propoxy, butoxy, trifluoromethoxy, trifluoroethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.), and the number of the substituents is preferably 1 to 3.

The substituent in the optionally substituted thiol group represented by R² includes the same as the "substituent in the optionally substituted hydroxyl group" as described above. Among them, it preferably includes:
(1) an optionally substituted alkyl group (e.g., a C₁₋₁₀ alkyl group such as methyl, ethyl, propyl, isopropyl, butyl, isobutyl, sec-butyl, tert-butyl, pentyl, isopentyl, neopentyl, hexyl, heptyl, octyl, nonyl and decyl, preferably a lower (C₁₋₆) alkyl group, or the like is exemplified); (2) an optionally substituted cycloalkyl group (e.g., C₃₋₇ cycloalkyl such as cyclopropyl, cyclobutyl, cyclopentyl, cyclohexyl and cycloheptyl,or the like is exemplified); (3) an optionally substituted aralkyl group (e.g., a phenyl-C₁₋₄ alkyl group (e.g., benzyl, phenethyl, etc.), or the like is exemplified); and (4) an optionally substituted aryl (e.g., phenyl, naphthyl, or the like is exemplified). The substituent which may be contained in (1) an optionally substituted alkyl group, (2) an optionally substituted cycloalkyl group, (3) an optionally substituted aralkyl group and (4) an optionally substituted aryl group as describe above includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5- or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

The substituent of the optionally substituted amino group represented by R² includes an amino group which may contain 1 or 2 of the same substituents as in the "optionally substituted hydroxyl group" as described above. Among them, it preferably includes:
(1) an optionally substituted alkyl group (e.g., a C₁₋₁₀ alkyl group such as methyl, ethyl, propyl, isopropyl, butyl, isobutyl, sec-butyl, tert-butyl, pentyl, isopentyl, neopentyl, hexyl, heptyl, octyl, nonyl, and decyl, preferably a lower (C₁₋₆) alkyl group, or the like is exemplified); (2) an optionally substituted cycloalkyl group (e.g., a C₃₋₇ cycloalkyl group such as cyclopropyl, cyclobutyl, cyclopentyl, cyclohexyl and cycloheptyl, or the like is exemplified); (3) an optionally substituted alkenyl group (e.g., a C₂₋₁₀ alkeny group such as allyl, crotyl, 2-pentenyl and 3-hexenyl, preferably a lower (C₂₋₆) alkenyl group, or the like is exemplified); (4) an optionally substituted cycloalkenyl group (e.g., a C₃₋₇ cycloalkenyl group such as 2-cyclopentenyl, 2-cyclohexenyl, 2-cyclopentenylmethyl and 2-cyclohexenylmethyl, or the like is exemplified); (5) a formyl group or an optionally substituted acyl group (e.g., a C₂₋₄ alkanoyl group such as acetyl, propionyl, butyryl and isobutyryl, and a C₁₋₄ alkylsulfonyl group (e.g., methanesulfonyl and ethanesulfonyl), or the like is exemplified); and (6) an optionally substituted aryl group (e.g., phenyl, naphthyl, or the like is exemplified). The substituent which may be contained in (1) an optionally substituted alkyl group, (2) an optionally substituted cycloalkyl group, (3) an optionally substituted alkenyl group, (4) an optionally substituted cycloalkenyl group, (5) an optionally substituted acyl group and (6) an optionally substituted aryl group as describe above, includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5- or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

In the optionally substituted amino group represented by R², the substituents of the amino group may bond each other to form a cyclic amino group (e.g., a cyclic amino group that is formed by removing one hydrogen atom from the nitrogen atom which constitutes a 5- or 6-membered ring such as tetrahydropyrrole, piperazine, piperidine, morpholine, thiomorpholine, pyrrole and imidazole, and has a bond on the nitrogen atom). The cyclic amino group may contain substituents, and the substituents include a halogen atom (e.g., fluorine, chlorine, bromine, iodine, etc.), nitro, cyano, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5- or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

The optionally substituted acyl group represented by R² includes the groups which (1) hydrogen; (2) an optionally substituted alkyl group (e.g., a C₁₋₁₀ alkyl group such as methyl, ethyl, propyl, isopropyl, butyl, isobutyl, sec-butyl, tert-butyl, pentyl, isopentyl, neopentyl, hexyl, heptyl, octyl, nonyl and decyl, preferably a lower (C₁₋₆) alkyl group, or the like is exemplified); (3) an optionally substituted cycloalkyl group (e.g., a C₃₋₇ cycloalkyl group such as cyclopropyl, cyclobutyl, cyclopentyl, cyclohexyl and cycloheptyl, or the like is exemplified); (4) an optionally substituted alkenyl group (e.g., a C₂₋₁₀ alkenyl group such as allyl, crotyl, 2-pentenyl and 3-hexenyl, preferably a lower (C₂₋₆) alkenyl group, or the like is exemplified); (5) an optionally substituted cycloalkenyl group (e.g., a C₃₋₇ cycloalkenyl group such as 2-cyclopentenyl, 2-cyclohexenyl, 2-cyclopentenylmethyl and 2-cyclohexenylmethyl, or the like is exemplified); (6) an optionally substituted 5- to 6-membered monocycle aromatic group (e.g., phenyl, pyridyl or the like is exemplified); or the like is bonded with the carbonyl group or the sulfonyl group, (e.g., acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl, etc.). The substituent which may be contained in (2) an optionally substituted alkyl group, (3) an optionally substituted cycloalkyl group, (4) an optionally substituted alkenyl group, (5) an optionally substituted cycloalkenyl group and (6) an optionally substituted 5- or 6-membered monocycle aromatic group as describe above, includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5- or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

The optionally esterified carboxyl group represented by R² includes the groups which (1) hydrogen; (2) an optionally substituted alkyl group (e.g., a C₁₋₁₀ alkyl group such as methyl, ethyl, propyl, isopropyl, butyl, isobutyl, sec-butyl, tert-butyl, pentyl, isopentyl, neopentyl, hexyl, heptyl, octyl, nonyl and decyl, preferably a lower (C₁₋₆) alkyl group, or the like is exemplified); (3) an optionally substituted cycloalkyl group (e.g., a C₃₋₇ cycloalkyl group such as cyclopropyl, cyclobutyl, cyclopentyl, cyclohexyl and cycloheptyl, or the like is exemplified); (4) an optionally substituted alkenyl group (e.g., a C₂₋₁₀ alkenyl group such as allyl, crotyl, 2-pentenyl and 3-hexenyl, preferably a lower (C₂₋₆) alkenyl group, or the like is exemplified); (5) an optionally substituted cycloalkenyl group (e.g., a C₃₋₇ cycloalkenyl group such as 2-cyclopentenyl, 2-cyclohexenyl, 2-cyclopentenylmethyl and 2-cyclohexenylmethyl, or the like is exemplified); (6) an optionally substituted aryl group (e.g., phenyl, naphthyl, etc.); or the like is bonded with a carbonyloxy group, preferably includes a carboxyl group, a lower (C₁₋₆) alkoxycarbonyl group, an aryloxycarbonyl group (e.g., methoxycarbonyl, ethoxycarbonyl, propoxycarbonyl, phenoxycarbonyl, naphthoxycarbonyl, etc.). The substituent which may be contained in (2) an optionally substituted alkyl group, (3) an optionally substituted cycloalkyl group, (4) an optionally substituted alkenyl group, (5) an optionally substituted cycloalkenyl group and (6) an optionally substituted aryl group as describe above, includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5-or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

R² preferably includes, in particular, a halogen atom, a cyano group, a hydroxyl group, a nitro group, an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, pyrrolidinocarbonyl, piperidinocarbonyl, morpholinocarbonyl, thiomorpholinocarbonyl, etc.), an optionally halogenated or lower-(C₁₋₄) alkoxylated lower (C₁₋₄) alkyl group (e.g., methyl, ethyl, t-butyl, trifluoromethyl, methoxymethyl, ethoxymethyl, propoxymethyl, butoxymethyl, methoxyethyl, ethoxyethyl, propoxyethyl, butoxyethyl, etc.), a lower (C₁₋₄) alkyl group which may be substituted with a hydroxyl group or a cyano group (e.g., hydroxy C₁₋₄ alkyl, cyano C₁₋₄ alkyl, etc.), a lower (C₁₋₄) alkyl group which may be substituted with an optionally esterified or amidated carboxyl group (e.g., carboxyl C₁₋₄ alkyl, C₁₋₄ alkoxycarbonyl C₁₋₄ alkyl, carbamoyl C₁₋₄ alkyl, mono-C₁₋₄ alkylcarbamoyl C₁₋₄ alkyl, di-C₁₋₄ alkylcarbamoyl C₁₋₄ alkyl, pyrrolidinocarbonyl C₁₋₄ alkyl, piperidinocarbonyl C₁₋₄ alkyl, morpholinocarbonyl C₁₋₄ alkyl, thiomorpholinocarbonyl C₁₋₄ alkyl, etc.), an optionally halogenated and lower-(C₁₋₄) alkoxylated lower (C₁₋₄) alkoxy group (e.g., methoxy, ethoxy, propoxy, butoxy, t-butoxy, trifluoromethoxy, methoxymethoxy, ethoxymethoxy, propoxymethoxy, butoxymethoxy, methoxyethoxy, ethoxyethoxy, propoxyethoxy, butoxyethoxy, methoxypropoxy, ethoxypropoxy, propoxypropoxy, butoxypropoxy, etc.), a halogen atom (e.g., fluorine, chlorine, etc.), a nitro group, a cyano group, an amino group which may be substituted with 1 or 2 of a lower (C₁₋₄) alkyl group, formyl or a lower (C₂₋₄) alkanoyl group (e.g., amino, methylamino, dimethylamino, formylamino, acetylamino, etc.) and a 5- or 6-membered cyclic amino group (e.g, 1-pyrrolidinyl, 1-piperazinyl, 1-piperidinyl, 4-morpholino, 4-thiomorpholino, 1-imidazolyl, 4-tetrahydropyranyl, etc.). For R², more particularly preferred is an optionally halogenated lower (C₁₋₄) alkyl group.

Definitions of each of the substituents represented by R^{2'} are the same as in R².

A "5- or 6-membered cyclic ring" in an "optionally substituted 5- or 6-membered cyclic ring group" represented by R³ includes a group that is formed by removing one hydrogen atom from a 6-membered aromatic hydrocarbon such as benzene, a 5- to 6-membered aliphatic hydrocarbon such as cyclopentane, cyclohexane, cyclopentene, cyclohexene, cyclopentadiene, and cyclohexadiene, a 5- to 6-membered aromatic heterocycle containing 1 to 4 heteroatoms of 1 to 2 kinds selected from a nitrogen atom, a sulfur atom and an oxygen atom, such as furan, thiophene, pyrrole, imidazole, pyrazole, thiazole, oxazole, isothiazole, isoxazole, tetrazole, pyridine, pyrazine, pyrimidine, pyridazine and triazole, a 5- to 6-membered non-aromatic heterocycle containing 1 to 4 heteroatoms of 1 to 2 kinds selected from a nitrogen atom, a sulfur atom and an oxygen atom, such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, and tetrahydrothiopyran, or the like. Among them, as for the "5- to 6-membered cyclic ring" is preferably benzene, furan, thiophene, pyridine, cyclopentane, cyclohexane, pyrrolidine, piperidine, piperazine, morpholine, thiomorpholine, tetrahydropyran (preferably, a 6-membered cyclic ring) or the like, particularly benzene.

The substituent which may be contained in the "5- or 6-membered cyclic ring" of the "optionally substituted 5-or 6-membered cyclic ring group" represented by R³ includes, for example, a halogen atom, nitro, cyano, optionally substituted alkyl, optionally substituted cycloalkyl, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group.

Halogen as the substituent of R³ includes fluorine, chlorine, bromine and iodine, and in particular preferably fluorine and chlorine.

An alkyl group in the optionally substituted alkyl group as the substituent of R³ includes a straight-chain or branched C₁₋₁₀ alkyl group, for example, a C₁₋₁₀ alkyl group such as methyl, ethyl, propyl, isopropyl, butyl, isobutyl, sec-butyl, tert-butyl, pentyl, isopentyl, neopentyl, hexyl, heptyl, octyl, nonyl and decyl, preferably a lower C₁₋₆ alkyl group. The substituent in the optionally substituted alkyl group includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5- or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

The cycloalkyl group in the optionally substituted cycloalkyl group as the substituent of R³ includes, for example, a C₃₋₇ cycloalkyl group such as cyclopropyl, cyclobutyl, cyclopentyl, cyclohexyl and cycloheptyl. The substituent in the optionally substituted cycloalkyl group includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5-or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

The substituent in the optionally substituted hydroxyl group as the substituent of R³ includes (1) an optionally substituted alkyl group (e.g., a C₁₋₁₀ alkyl group such as methyl, ethyl, propyl, isopropyl, butyl, isobutyl, sec-butyl, tert-butyl, pentyl, isopentyl, neopentyl, hexyl, heptyl, octyl, nonyl and decyl, preferably a lower (C₁₋₆) alkyl group, or the like is exemplified); (2) a cycloalkyl group that may be substituted and may contain heteroatoms (e.g., C₃₋₇ cycloalkyl such as cyclopropyl, cyclobutyl, cyclopentyl, cyclohexyl and cycloheptyl; a saturated 5- to 6-membered heterocyclic group containing 1 to 2 heteroatoms such as tetrahydrofuranyl, tetrahydrothienyl, pyrrolidinyl, pyrazolidinyl, piperidyl, piperazinyl, morpholinyl, thiomorpholinyl, tetrahydropyranyl and tetrahydrothiopyranyl (preferably, tetrahydropyranyl, etc.); or the like is exemplified); (3) an optionally substituted alkenyl group (e.g., a C₂₋₁₀ alkenyl group such as allyl, crotyl, 2-pentenyl and 3-hexenyl, preferably a lower (C₂₋₆) alkenyl group, or the like is exemplified); (4) an optionally substituted cycloalkenyl group (e.g., a C₃₋₇ cycloalkenyl group such as 2-cyclopentenyl, 2-cyclohexenyl, 2-cyclopentenylmethyl and 2-cyclohexenylmethyl, or the like is exemplified); (5) an optionally substituted aralkyl group (e.g., phenyl-C₁₋₄ alkyl (e.g., benzyl, phenethyl, etc.) or the like is exemplified); (6) a formyl group or an optionally substituted acyl group (e.g., a C₂₋₄ alkanoyl group such as acetyl, propionyl, butyryl and isobutyryl, and a C₁₋₄ alkylsulfonyl group such as methanesulfonyl and ethanesulfonyl, or the like is exemplified); and (7) an optionally substituted aryl group (e.g., phenyl, naphthyl, or the like is exemplified). The substituent which may be contained in (1) an optionally substituted alkyl group, (2) an optionally substituted cycloalkyl group, (3) an optionally substituted alkenyl group, (4) an optionally substituted cycloalkenyl group, (5) an optionally substituted aralkyl group, (6) an optionally substituted acyl group, and (7) an optionally substituted aryl group as described above, includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5- or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkyl group (e.g., trifluoromethyl, methyl, ethyl, etc.), an optionally halogenated C₁₋₆ alkoxy group (e.g., methoxy, ethoxy, propoxy, butoxy, trifluoromethoxy, trifluoroethoxy, etc.; preferably an optionally halogenated C₁₋₄ alkoxy group), a formyl group, a C₂₋₄ alkanoyl group (e.g., acetyl, propionyl, etc.) and a C₁₋₄ alkylsulfonyl group (e.g., methanesulfonyl, ethanesulfonyl, etc.), a 5-to 6-membered aromatic heterocycle that may be substituted [for example, an 5- to 6-membered aromatic heterocycle containing 1 to 4 heteroatoms of 1 to 2 kinds selected from a nitrogen atom, a sulfur atom and an oxygen atom, such as furan, thiophene, pyrrole, imidazole, pyrazole, thiazole, oxazole, isothiazole, isoxazole, tetrazole, pyridine, pyrazine, pyrimidine, pyridazine and triazole, etc.; the substituent that may be contained in the heterocycle, includes a halogen atom (e.g., fluorine, chlorine, bromine, iodine, etc.), a nitro group, a cyano group, a hydroxyl group, a thiol group, an amino group, a carboxyl group, an optionally halogenated C₁₋₄ alkyl group (e.g., trifluoromethyl, methyl, ethyl, etc.), a C₁₋₄ alkoxy group (e.g., methoxy, ethoxy, propoxy, butoxy, trifluoromethoxy, trifluoroethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.), and the number of the substituents is preferably 1 to 3]. The number of the substituents is preferably 1 to 3.

Further, the substituent in the optionally substituted thiol group as the substituent of R³ includes the same as the "substituents in the optionally substituted hydroxyl group as the substituent of R¹" as described above. Among them, it preferably includes:
(1) an optionally substituted alkyl group (e.g., a C₁₋₁₀ alkyl group such as methyl, ethyl, propyl, isopropyl, butyl, isobutyl, sec-butyl, tert-butyl, pentyl, isopentyl, neopentyl, hexyl, heptyl, octyl, nonyl and decyl, preferably a lower (C₁₋₆) alkyl group, or the like is exemplified); (2) an optionally substituted cycloalkyl group (e.g., C₃₋₇ cycloalkyl such as cyclopropyl, cyclobutyl, cyclopentyl, cyclohexyl and cycloheptyl, or the like is exemplified); (3) an optionally substituted aralkyl group (e.g., a phenyl-C₁₋₄ alkyl group (e.g., benzyl, phenethyl, etc.) or the like is exemplified); and (4) an optionally substituted aryl group (e.g., phenyl, naphthyl or the like is exemplified). The substituent which may be contained in (1) an optionally substituted alkyl group, (2) an optionally substituted cycloalkyl group, (3) an optionally substituted aralkyl group and (4) an optionally substituted aryl group as describe above, includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5- or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

The substituent of the optionally substituted amino group as the substituent of R³ includes an amino group which may contain 1 or 2 substituents of the same as the "substituent of the optionally substituted hydroxyl group as the substituent of R¹". Among them, it preferably includes:
(1) an optionally substituted alkyl group (e.g., a C₁₋₁₀ alkyl group such as methyl, ethyl, propyl, isopropyl, butyl, isobutyl, sec-butyl, tert-butyl, pentyl, isopentyl, neopentyl, hexyl, heptyl, octyl, nonyl and decyl, preferably a lower (C₁₋₆) alkyl group, or the like is exemplified); (2) an optionally substituted cycloalkyl group (e.g., a C₃₋₇ cycloalkyl group such as cyclopropyl, cyclobutyl, cyclopentyl, cyclohexyl and cycloheptyl, or the like is exemplified); (3) an optionally substituted alkenyl group (e.g., a C₂₋₁₀ alkenyl group such as allyl, crotyl, 2-pentenyl and 3-hexenyl, preferably a lower (C₂₋₆) alkenyl group, or the like is exemplified); (4) an optionally substituted cycloalkenyl group (e.g., a C₃₋₇ cycloalkenyl group such as 2-cyclopentenyl, 2-cyclohexenyl, 2-cyclopentenylmethyl and 2-cyclohexenylmethyl, or the like is exemplified); (5) a formyl group or an optionally substituted acyl group (e.g., a C₂₋₄ alkanoyl group such as acetyl, propionyl, butyryl and isobutyryl), a C₁₋₄ alkylsulfonyl group (e.g., methanesulfonyl and ethanesulfonyl), or the like is exemplified); and (6) an optionally substituted aryl group (e.g., phenyl, naphthyl, or the like is exemplified). The substituent which may be contained in (1) an optionally substituted alkyl group, (2) an optionally substituted cycloalkyl group, (3) an optionally substituted alkenyl group, (4) an optionally substituted cycloalkenyl group, (5) an optionally substituted acyl group and (6) an optionally substituted aryl group as describe above, includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5- or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

Further, in the optionally substituted amino group as the substituent of R³, the substituents of the amino group may bond each other to form a cyclic amino group (for example, a cyclic amino group that is formed by removing one hydrogen from the nitrogen atom constituting the ring of a 5- to 6-membered cyclic ring such as tetrahydropyrrole, piperazine, piperidine, morpholine, thiomorpholine, pyrrole and imidazole, and has a bond on the nitrogen atom). The cyclic amino group may contain a substituent, and the substituent includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5- or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

The optionally substituted acyl group as the substituent of R³ includes those which (1) hydrogen; (2) an optionally substituted alkyl group (e.g., a C₁₋₁₀ alkyl group such as methyl, ethyl, propyl, isopropyl, butyl, isobutyl, sec-butyl, tert-butyl, pentyl, isopentyl, neopentyl, hexyl, heptyl, octyl, nonyl and decyl, preferably a lower (C₁₋₆) alkyl group, or the like is exemplified); (3) an optionally substituted cycloalkyl group (e.g., a C₃₋₇ cycloalkyl group such as cyclopropyl, cyclobutyl, cyclopentyl, cyclohexyl and cycloheptyl, or the like is exemplified); (4) an optionally substituted alkenyl group (e.g., a C₂₋₁₀ alkenyl group such as allyl, crotyl, 2-pentenyl and 3-hexenyl, preferably a lower (C₂₋₆) alkenyl group, or the like is exemplified); (5) an optionally substituted cycloalkenyl group (e.g., a C₃₋₇ cycloalkenyl group such as 2-cyclopentenyl, 2-cyclohexenyl, 2-cyclopentenylmethyl and 2-cyclohexenylmethyl, or the like is exemplified); (6) a 5- to 6-membered monocycle aromatic group (for example, phenyl, pyridyl or the like is exemplified); or the like is bonded with the carbonyl group or the sulfonyl group, (for example, acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl or the like). The substituent which may be contained in (2) an optionally substituted alkyl group, (3) an optionally substituted cycloalkyl group, (4) an optionally substituted alkenyl group, (5) an optionally substituted cycloalkenyl group and (6) an optionally substituted aromatic group of a 5- or 6-membered monocycle as describe above, includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5-or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

The optionally esterified carboxyl group as the substituent of R³ includes those which (1) hydrogen; (2) an optionally substituted alkyl group (e.g., a C₁₋₁₀ alkyl group such as methyl, ethyl, propyl, isopropyl, butyl, isobutyl, sec-butyl, tert-butyl, pentyl, isopentyl, neopentyl, hexyl, heptyl, octyl, nonyl and decyl, preferably a lower (C₁₋₆) alkyl group, or the like is exemplified); (3) an optionally substituted cycloalkyl group (e.g., a C₃₋₇ cycloalkyl group such as cyclopropyl, cyclobutyl, cyclopentyl, cyclohexyl and cycloheptyl, or the like is exemplified); (4) an optionally substituted alkenyl group (e.g., a C₂₋₁₀ alkenyl group such as allyl, crotyl, 2-pentenyl and 3-hexenyl, preferably a lower (C₂₋₆) alkenyl group, or the like is exemplified); (5) an optionally substituted cycloalkenyl group (e.g., a C₃₋₇ cycloalkenyl group such as 2-cyclopentenyl, 2-cyclohexenyl, 2-cyclopentenylmethyl and 2-cyclohexenylmethyl, or the like is exemplified); (6) an optionally substituted aryl group (e.g., phenyl,naphthyl, etc.); or the like is bonded with a carbonyloxy group, preferably includes a carboxyl group, a lower (C₁₋₆) alkoxycarbonyl group and an aryloxycarbonyl group (e.g., methoxycarbonyl, ethoxycarbonyl, propoxycarbonyl, phenoxycarbonyl, naphthoxycarbonyl, etc.). The substituent which may be contained in (2) an optionally substituted alkyl group, (3) an optionally substituted cycloalkyl group, (4) an optionally substituted alkenyl group, (5) an optionally substituted cycloalkenyl group and (6) an optionally substituted aryl group as describe above, includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5-or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkoxy group (e.g., methoxy, ethoxy, propoxy, butoxy, trifluoromethoxy, trifluoroethoxy, etc.), an optionally halogenated C₁₋₄ alkoxy-C₁₋₄ alkoxy group (e.g., methoxymethoxy, methoxyethoxy, ethoxyethoxy, trifluoromethoxyethoxy, trifluoroethoxyethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

The aromatic group in the optionally substituted aromatic group as the substituent of R³ includes a 5- or 6-membered homo- or heterocyclic aromatic group such as phenyl, pyridyl, furyl, thienyl, pyrrolyl, imidazolyl, pyrazolyl, thiazolyl, oxazolyl, isothiazolyl, isoxazolyl, tetrazolyl, pyrazinyl, pyrimidinyl, pyridazinyl and triazolyl; and a condensed heterocyclic aromatic group such as benzofuran, indole, benzothiophene, benzoxazole, benzothiazole, indazole, benzimidazole, quinoline, isoquinoline, quinoxaline, phthalazine, quinazoline, cinnoline and imidazopyridine. The substituent in the aromatic group includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5- or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkyl group (e.g., trifluoromethyl, methyl, ethyl, etc.), an optionally halogenated C₁₋₄ alkoxy group (e.g., methoxy, ethoxy, propoxy, butoxy, trifluoromethoxy, trifluoroethoxy, etc.), a formyl group, a C₂₋₄ alkanoyl group (e.g., acetyl, propionyl, etc.) and a C₁₋₄ alkylsulfonyl group (e.g., methanesulfonyl, ethanesulfonyl, etc.). The number of the substituents is preferably 1 to 3.

Such a substituent of R³ may be substituted at any position of 1 to 4 (preferably, 1 to 2) of the same or different cyclic rings. Further, in the case where the "5-or 6-membered cyclic ring" of the "optionally substituted 5- or 6-membered cyclic ring" represented by R³ contains at least 2 substituents, two substituents among them may bond each other to form, for example, a lower (C₁₋₆) alkylene group (e.g., trimethylene, tetramethylene, etc.), a lower (C₁₋₆) alkyleneoxy group (e.g., -CH₂-O-CH₂-, -O-CH₂-CH₂-, -O-CH₂-CH₂-CH₂-, -O-CH₂-CH₂-CH₂-CH₂-, -O-C(CH₃) (CH₃)-CH₂-CH₂-, etc.), a lower (C₁₋₆) alkylene thio group (e.g., -CH₂-S-CH₂-, -S-CH₂-CH₂-, -S-CH₂-CH₂-CH₂-, -S-CH₂-CH₂-CH₂-CH₂-, -S-C(CH₃)(CH₃)-CH₂-CH₂-, etc.), a lower (C₁₋₆) alkylenedioxy group (e.g., -O-CH₂-O-, -O-CH₂-CH₂-O-, -O-CH₂-CH₂-CH₂-O-, etc.), a lower (C₁₋₆) alkylenedithio group (e.g., -S-CH₂-S-, -S-CH₂-CH₂-S-, -S-CH₂-CH₂-CH₂-S-, etc.), an oxy-lower (C₁₋₆) alkylene amino group (e.g., -O-CH₂-NH-, -O-CH₂-CH₂-NH-, etc.), an oxy-lower (C₁₋₆) alkylene thio group (e.g., -O-CH₂-S-, -O-CH₂-CH₂-S-, etc.), a lower (C₁₋₆) alkylene amino group (e.g., -NH-CH₂-CH₂-, -NH-CH₂-CH₂-CH₂-, etc.), a lower (C₁₋₆) alkylene diamino group (e.g., -NH-CH₂-NH- and -NH-CH₂-CH₂-NH-), a thia-lower (C₁₋₆) alkylene amino group (e.g., - S-CH₂-NH-, -S-CH₂-CH₂-NH-, etc.), a lower (C₂₋₆) alkenylene group (e.g., -CH₂-CH=CH-, -CH₂-CH₂-CH=CH-, -CH₂-CH=CH-CH₂-, etc.) and a lower (C₄₋₆) alkadienylene group (e.g., -CH=CH-CH=CH-, etc.).

In addition, the divalent group formed by bonding two substituents of R³ each other may contain the same 1 to 3 substituents as those which may be contained in the "5- or 6-membered cyclic ring" of the "optionally substituted 5-or 6-membered cyclic ring" represented by R³ [a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified or amidated carboxyl group or an optionally substituted aromatic group).

The substituent which may be contained in the "5- or 6-membered cyclic ring" of the "optionally substituted 5-or 6-membered cyclic ring" represented by R³, includes in particular an optionally halogenated or lower-(C₁₋₄) alkoxylated lower (C₁₋₄) alkyl group (e.g., methyl, ethyl, t-butyl, trifluoromethyl, methoxymethyl, ethoxymethyl, propoxymethyl, butoxymethyl, methoxyethyl, ethoxyethyl, propoxyethyl, butoxyethyl, etc.), an optionally halogenated or lower-(C₁₋₄) alkoxylated lower (C₁₋₄) alkoxy group (e.g., methoxy, ethoxy, propoxy, butoxy, t-butoxy, trifluoromethoxy, methoxymethoxy, ethoxymethoxy, propoxymethoxy, butoxymethoxy, methoxyethoxy, ethoxyethoxy, propoxyethoxy, butoxyethoxy, methoxypropoxy, ethoxypropoxy, propoxypropoxy, butoxypropoxy, etc.), a halogen atom (e.g., fluorine, chlorine, etc.), a nitro group, a cyano group, an amino group which may be substituted with 1 or 2 lower (C₁₋₄) alkyl groups, formyl or lower (C₂₋₄) alkanoyl (e.g., amino, methylamino, dimethylamino, formylamino and acetylamino), and a 5- or 6-membered cyclic amino group (e.g., 1-pyrrolidinyl, 1-piperadinyl, 1-piperidinyl, 4-morpholino, 4-thiomorpholino, 1-imidazolyl and 4-tetrahydropyranyl).

The "divalent group comprising a linear part constituted of 1 to 4 atoms" represented by X includes, for example, -(CH₂)_{a'}- [a' is an integer of 1 to 4 (preferably 1 or 2)], -(CH₂)_{b'}-X¹- [b' is an integer of 0 to 3 (preferably 0 or 1), X¹ is an optionally substituted imino group (e.g., an imino group which may be substituted with lower (C₁₋₆) alkyl, lower (C₃₋₇) cycloalkyl, formyl, lower (C₂₋₇) alkanoyl and lower (C₁₋₆) alkoxycarbonyl), a carbonyl group, an oxygen atom or an optionally oxidized sulfur atom (e.g., -S(O)ₘ- (m is an integer of 0 to 2) or the like)], -CH=CH-, -C≡C-, -CO-NH- and -SO₂-NH-. Such group may bond with a condensed ring by either of a bond on the left and a bond on the right, but preferably it binds with a condensed ring by a bond on the right.

X is preferably a bond, -(CH₂)_{b'}-O- [b' is an integer of 0, 1 or 2 (preferably 0 or 1)] and -C≡C-, and more preferably a bond.

The lower alkyl group of the "optionally substituted lower alkyl group" represented by R⁴ as described above includes, for example, a C₁₋₆ alkyl group such as methyl, ethyl, propyl, isopropyl, butyl, isobutyl, sec-butyl, tert-butyl, pentyl, isopentyl, neopentyl and hexyl.

The lower alkoxy group of the "optionally substituted lower alkoxy group" represented by R⁴ as described above includes a C₁₋₆ alkoxy group such as methoxy, ethoxy, propoxy and butoxy.

The substituent which may be contained in the "optionally substituted lower alkyl group" and "the optionally substituted lower alkoxy group" includes, for example, a halogen atom (e.g., fluorine, chlorine, bromine and iodine), a hydroxyl group, an amino group, a mono- (lower alkyl) amino group, a di-(lower alkyl) amino group and a lower alkanoyl group.

The lower alkyl which is contained in the mono-(lower alkyl) amino group and the di-(lower alkyl) amino group includes, for example, the same lower alkyl group as in the "optionally substituted lower alkyl group" represented by R⁴ as described above.

The lower alkanoyl group includes, for example, a C₂₋₆ alkanoyl group such as acetyl, propionyl, butyryl and isobutyryl.

The "halogen atoms" represented by R⁴ as described above includes fluorine, chlorine, bromine and iodine.

Among them, R⁴ is preferably an optionally substituted lower C₁₋₆ alkyl group, preferably a halogen atom, and in particular preferably an optionally substituted methyl group and a halogen atom.

The "hydrocarbon group" of the "optionally substituted hydrocarbon group" represented by R⁵ includes, for example, (1) an alkyl group (e.g., a C₁₋₁₀ alkyl group such as methyl, ethyl, propyl, isopropyl, butyl, isobutyl, sec-butyl, tert-butyl, pentyl, isopentyl, neopentyl, hexyl, heptyl, octyl, nonyl and decyl, preferably a lower (C₁₋₆) alkyl group, more preferably a lower (C₁₋₄) alkyl group, or the like is exemplified); (2) a cycloalkyl group (e.g., a C₃₋₇ cycloalkyl group such as cyclopropyl, cyclobutyl, cyclopentyl, cyclohexyl and cycloheptyl, or the like is exemplified); (3) an alkenyl group (e.g., a C₂₋₁₀ alkenyl group such as allyl, crotyl, 2-pentenyl and 3-hexenyl, preferably a lower (C₂₋₆) alkenyl group, or the like is exemplified); (4) a cycloalkenyl group (e.g., a C₃₋₇ cycloalkenyl group such as 2-cyclopentenyl, 2-cyclohexenyl, 2-cyclopentenylmethyl and 2-cyclohexenylmethyl, or the like is exemplified); (5) an alkynyl group (e.g., a C₂₋₁₀ alkynyl group such as ethynyl, 1-propynyl, 2-propynyl, 1-butynyl, 2-pentynyl and 3-hexynyl, preferably a lower a C₂₋₆ alkynyl group, or the like is exemplified), (6) an aralkyl group (e.g., phenyl-C₁₋₄ alkyl (e.g., benzyl, phenethyl, etc.), or the like is exemplified), (7) an aryl group (e.g., phenyl, naphthyl, or the like is exemplified), and (8) a cycloalkyl-alkyl group (e.g., a C₃₋₇ cycloalkyl-C₁₋₄ alkyl group such as cyclopropylmethyl, cyclobutylmethyl, cyclopentylmethyl, cyclohexylmethyl and cycloheptyl methyl, or the like is exemplified). The substituent which may be contained in (1) an alkyl group, (2) a cycloalkyl group, (3) an alkenyl group, (4) a cycloalkenyl group, (5) an alkynyl group, (6) an aralkyl group, (7) an aryl group and (8) a cycloalkyl-alkyl group as described above, includes a halogen atom (e.g., fluorine, chlorine, bromine, iodine, etc.), a nitro group, a cyano group, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, a 5-or 6-membered cyclic amino group such as tetrahydropyrrole, piperazine, piperidine, morpholine, thiomorpholine, pyrrole and imidazole, etc.), an optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, etc.), an optionally halogenated C₁₋₄ alkyl group (e.g., trifluoromethyl, methyl, ethyl, etc.), an optionally halogenated C₁₋₄ alkoxy group (e.g., methoxy, ethoxy, propoxy, butoxy, trifluoromethoxy, trifluoroethoxy, etc.), a C₁₋₄ alkylenedioxy group (e.g., -O-CH₂-O, -O-CH₂-CH₂-O-, etc.), an optionally substituted sulfonamide group [e.g, a group formed by bonding an optionally substituted amino group (e.g., amino, mono-C₁₋₄ alkylamino, di-C₁₋₄ alkylamino, a 5- or 6-membered cyclic amino group such as tetrahydropyrrole, piperazine, piperidine, morpholine, thiomorpholine, pyrrole and imidazole, etc.) with -SO₂-, etc.], a formyl group, a C₂₋₄ alkanoyl group (e.g., acetyl and propionyl), a C₁₋₄ alkylsulfonyl group (e.g., methanesulfonyl and ethanesulfonyl) and an optionally substituted heterocyclic group. The number of the substituents is preferably 1 to 3.

The "optionally substituted heterocyclic group" represented by R⁵ includes, for example, a group formed by removing one hydrogen atom from an aromatic heterocycle or a non-aromatic heterocycle. The aromatic heterocycle includes a 5- or 6-membered, aromatic heterocycle 1 to 4 of 1 or 2 kinds of hetero atoms selected from a nitrogen atom, a sulfur atom and an oxygen atom, such as furan, thiophene, pyrrole, imidazole, pyrazole, thiazole, oxazole, isothiazole, isoxazole, tetrazole, pyridine, pyrazine, pyrimidine, pyridazine, triazole, oxadiazole, thiadiazole, etc., and the non-aromatic heterocycle includes, for example, a 5- or 6-membered non-aromatic heterocycle containing 1 to 4 of 1 or 2 kinds of hetero atoms selected from a nitrogen atom, a sulfur atom and an oxygen atom, and a non-aromatic heterocycle which a part or all of the bonds of the above-described aromatic heterocycle is saturated bonds, such as tetrahydrofuran, tetrahydrothiophene, dioxolane, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran and tetrahydropyran (preferably an aromatic heterocycle such as pyrazole, thiazole, oxazole and tetrazole).

The substitutent of the "optionally substituted sulfonyl group" represented by R⁵ includes the same substituents as that in the "optionally substituted thiol group" described as the substituent of R³.

The "optionally esterified carboxyl group" and the "optionally substituted acyl group" represented by R⁵ include the same substituents as those described as the substituent of R³.

The preferable examples of R⁵ includes a hydrogen atom, an optionally substituted hydrocarbon group and an optionally substituted acyl group, more preferably a C₁₋₆ alkyl group, a C₁₋₄ alkylsulfonyl group, a formyl group and a C₂₋₅ alkanoyl group, even more preferably a C₁₋₄ alkyl group, a formyl group, a C₂₋₅ alkanoyl group, and even more preferably a propyl group or an isobutyl group.

The halogen atom represented by Y includes a chlorine atom and a bromine atom. A lower alkyl group represented by R⁹ in -OSO₂-R⁹ which is represented by Y includes a C₁₋₆ alkyl group such as methyl, ethyl, propyl, isopropyl, butyl, isobutyl, sec-butyl, t-butyl, pentyl, isopentyl, neopentyl, 1-ethylpropyl, hexyl, isohexyl, 1,1-dimethylbutyl, 2,2-dimethylbutyl, 3,3-dimethylbutyl and 2-ethylbutyl. The aryl group in the optionally substituted aryl group represented by R⁹ includes, for example, phenyl and naphthyl, and the substituent in the optionally substituted aryl group includes, for example, a C₁₋₆ alkyl group (e.g., methyl, ethyl, propyl, isopropyl, butyl, isobutyl, sec-butyl, tert-butyl, pentyl, etc.), a C₁₋₆ alkoxy group (e.g., methoxy, ethoxy, propoxy, isopropoxy, butoxy, isobutoxy, sec-butoxy, tert-butoxy, etc.), a halogen atom (e.g., chlorine, bromine, iodine, fluorine, etc.), a nitro group and a cyano group. A preferable example of the group represented by the formula -OSO₂-R⁹ includes a methanesulfonyloxy group and a p-toluenesulfonyloxy group.

m is an integer of 1 to 5, preferably 2 to 4.

n is an integer of 0 to 3, preferably 0 or 1.

^{*1} and ^{*2} mean that the atom to which ^{*1} or ^{*2} is attached is an asymmetric atom, respectively.

R⁶ represents a methyl group, a phenyl group, a 4-methylphenyl group or α-naphthyl group. R⁷ represents a chlorine atom or a nitro group.

The lower alkyl group in the optionally substituted lower alkyl group represented by R⁸ includes, for example, a halogen atom (e.g., fluorine, chlorine, bromine and iodine), a hydroxyl group, an amino group, a mono-(lower alkyl) amino group, a di-(lower alkyl) amino group and a lower alkanoyl group.

The aryl group in the optionally substituted aryl group represented by R⁸ includes, for example, a C₆₋₁₀ aryl group such as a phenyl group and a naphthyl group, and the substituent in the optionally substituted aryl group includes, for example, a halogen atom (e.g., fluorine, chlorine, bromine and iodine), a hydroxyl group, an amino group, a mono-(lower alkyl) amino group, a di-(lower alkyl) amino group and a lower alkanoyl group.

The aralkyl group in the optionally substituted aralkyl group represented by R⁸ includes, for example, a C₇₋₁₀ aralkyl group such as a benzyl group and a phenethyl group, and the substituent in the optionally substituted aralkyl group includes, for example, a halogen atom (e.g., fluorine, chlorine, bromine and iodine), a hydroxyl group, an amino group, a mono-(lower alkyl) amino group, a di-(lower alkyl) amino group and a lower alkanoyl group.

The optionally substituted lower alkyl group, the optionally substituted aryl group and the optionally substituted aralkyl group represented by R¹⁰ in -OR¹⁰ which is represented by R⁸, includes those described as the optionally substituted lower alkyl group, the optionally substituted aryl group and the optionally substituted aralkyl group represented by R⁸, respectively.

### Best Mode for Carrying Out the Invention

Hereinafter, each of the reaction processes of the present invention will be described in detail.

As described below, each of the compounds represented by the formulas (I), (II), (III), (IV), (V), (VI), (VII), (VIII), (IX), (X), (XIa) and (XIb) can form a salt thereof. The salts include, for example, a salt with an inorganic base, a salt with an organic base, a salt with an inorganic acid, a salt with an organic acid, and a salt with a basic or acidic amino acid. A preferable example of a salt with an inorganic base includes, for example, an alkali metal salt such as a sodium salt and a potassium salt; an alkaline earth metal salt such as a calcium salt and a magnesium salt; an aluminum salt; and an ammonium salt. A preferable example of a salt with an organic base includes, for example, salts with trimethylamine, triethylamine, pyridine, picoline, ethanolamine, diethanolamine, triethanolamine, dicyclohexylamine and N,N'-dibenzylethylenediamine. A preferable example of a salt with an inorganic acid includes, for example, a salt with hydrochloric acid, hydrobromic acid, nitric acid, sulfuric acid and phosphoric acid. A preferable example of a salt with an organic acid includes, for example, a salt with formic acid, acetic acid, trifluoroacetic acid, fumaric acid, oxalic acid, tartaric acid, maleic acid, citric acid, succinic acid, malic acid, methanesulfonic acid, benzenesulfonic acid and p-toluenesulfonic acid. A preferable example of a salt with a basic amino acid includes, for example, a salt with arginine, lysine, and ornithine, and a preferable example of a salt with an acidic amino acid includes, for example, a salt with asparagic acid, and glutamic acid.

The compound (I) thus obtained can be isolated and purified by a well-known separation-purification means, for example, concentration, concentration under reduced pressure, solvent extraction, crystallization, recrystallization, solution conversion and chromatography.

Hereinafter, a compound represented by the formula (I) and a salt thereof are simply referred to as Compound (I). Similarly, a compound represented by the formula (II), a compound represented by the formula (III), a compound represented by the formula (IV), a compound represented by the formula (V), a compound represented by the formula (VI), a compound represented by the formula (VII), a compound represented by the formula (VIII), a compound represented by the formula (IX), a compound represented by the formula (X), a compound represented by the formula (XIa) and a compound represented by the formula (XIb), including salts thereof, are simply referred to as Compounds (I), (II),(III), (IV), (V), (VI), (VII), (VIII), (IX), (X), (XIa) and (XIb), respectively. In addition, a compound represented by the formula (XIIa), a compound represented by the formula (XIIb), and a compound represented by the formula (XIIc) are also referred to as the compound (XIIa), the compound (XIIb) and the compound (XIIc), respectively.

These compounds can be isolated by a general isolation-production means after the reaction in each process, but they may be also used for the next reaction without isolation.

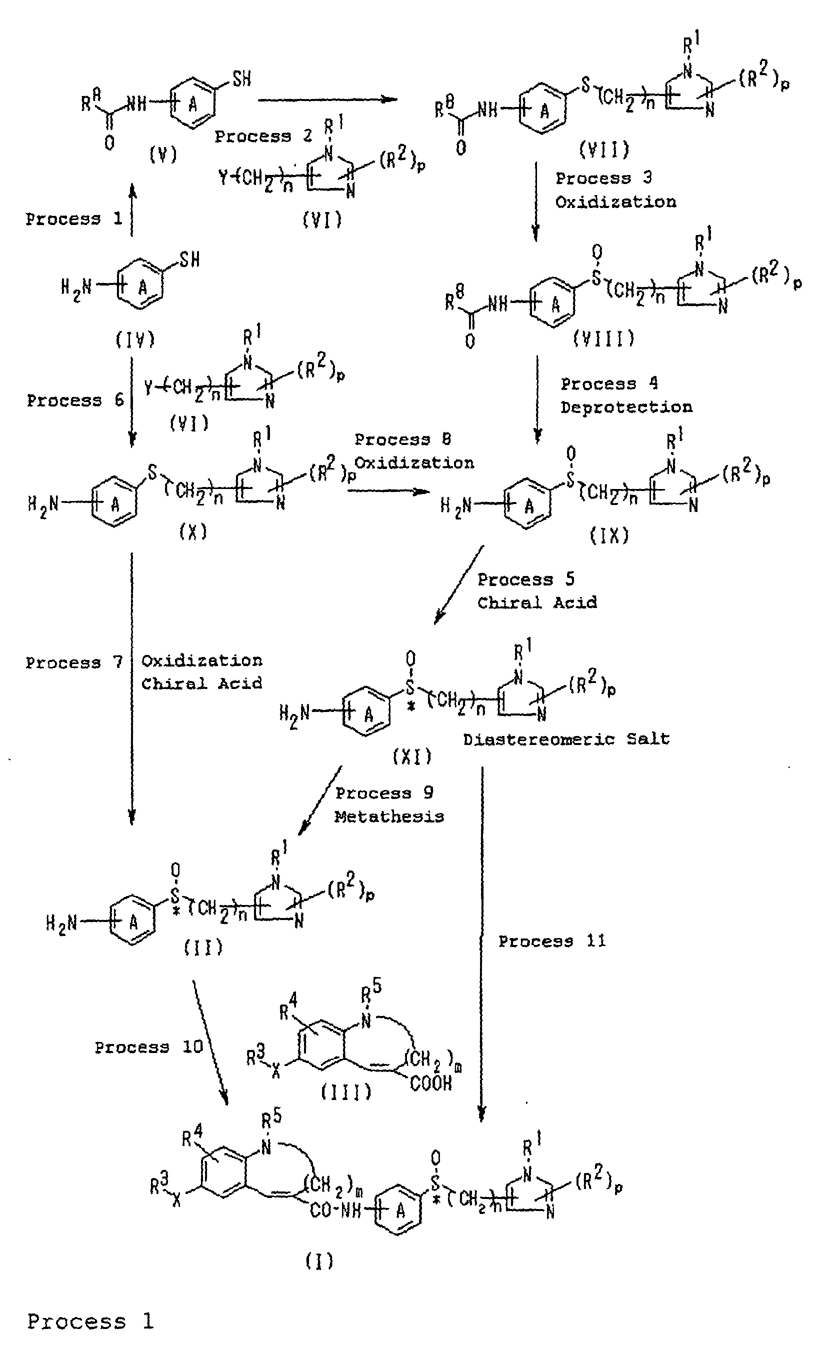

The compound (V) can be prepared by reacting the compound (IV), for example, with an acylating agent (e.g., acid chloride, acid bromide, a mixed acid anhydride and active ester). This reaction is performed in an appropriate solvent. The solvent includes, for example, aromatic hydrocarbons (e.g., benzene, toluene and xylene), ethers (e.g., dioxane, tetrahydrofuran (THF) and dimethoxyethane), esters (e.g., ethyl acetate), nitriles (e.g., acetonitrile), ketones (e.g., acetone, 2-butanone, 2-pentanone, 3-pentanone, 2-hexanone, 3-hexanone and methyl isobutyl ketone), tertiary amines (e.g., pyridine), N,N-dimethylformamide (DMF), dimethylsulfoxide (DMSO), N-methylpyrrolidone (NMP), halogenated hydrocarbons (e.g., chloroform, dichloromethane, 1,2-dichloroethane and 1,1,2,2-tetrachloroethane) and a mixed solvent thereof. The amount of the acylating agent used is preferably about 1 to 5 mole equivalents, based on the compound (IV). In addition, this reaction may be also performed in the presence of a base. The base includes an alkali metal salt (e.g., potassium hydroxide, sodium hydroxide, potassium carbonate, sodium carbonate, potassium hydrogen carbonate and sodium hydrogen carbonate), amines (e.g., trimethylamine, triethylamine, diisopropyl ethylamine, N-methylmorpholine, 1,8-diazabicyclo[5.4.0]-7-undecene (DBU) and 1,4-diazabicyclo[2,2,2]octane (DABCO)) and aromatic amines (e.g., N,N-dimethylaminopyridine, N,N-diethylamino pyridine, pyridine, 4-dimethylaminopyridine, picoline and quinoline). This reaction is performed generally at -20°C to 200°C, preferably at about -10°C to 150°C.

### Process 2

The compound (VII) can be prepared by reacting the compound (V) with the compound (VI). The reaction is performed in an appropriate solvent. The solvent includes, for example, aromatic hydrocarbons (e.g., benzene, toluene and xylene), ethers (e.g., dioxane, tetrahydrofuran (THF) and dimethoxyethane), alcohols (e.g., methanol, ethanol, propanol, isopropanol, butanol, isobutanol, sec-butanol and tert-butanol), esters (e.g., ethyl acetate), nitriles (e.g., acetonitrile), ketones (e.g., acetone, 2-butanone, 2-pentanone, 3-pentanone, 2-hexanone, 3-hexanone and methyl isobutyl ketone), pyridine, N,N-dimethylformamide (DMF), dimethylsulfoxide (DMSO), N-methylpyrrolidone (NMP), halogenated hydrocarbons (e.g., chloroform, dichloromethane, 1,2-dichloroethane and 1,1,2,2-tetrachloroethane), water and a mixed solvent thereof. This reaction may be performed in the presence of a base. The base includes an alkali metal salt (e.g., potassium hydroxide, sodium hydroxide, potassium carbonate, sodium carbonate, potassium hydrogen carbonate and sodium hydrogen carbonate), a metal hydride (e.g., potassium hydride, sodium hydride and calcium hydride), amines (e.g., trimethylamine, triethylamine, diisopropyl ethylamine, N-methylmorpholine, 1,8-diazabicyclo[5.4.0]-7-undecene (DBU), 1,4-diazabicyclo[2,2,2]octane (DABCO)) and aromatic amines (e.g., N,N-dimethylaminopyridine, N,N-diethylaminopyridine, pyridine, 4-dimethylaminopyridine, picoline and quinoline). The amount of the compound (VI) used is about 1 to 5 mole equivalents, preferably about 1 to 3 mole equivalents, based on Compound (V). In addition, the amount of the base used is preferably about 1 to 5 mole equivalents, based on the compound (V). This reaction is performed generally at - 20°C to 200°C, preferably at about -10°C to 150°C.

### Process 3

The compounds (VIII) can be prepared by oxidizing the compound (VII). The reaction is performed in an appropriate solvent. The solvent includes, for example, carbohydrates (e.g., benzene, toluene and xylene), ethers (e.g., dioxane, tetrahydrofuran (THF) and dimethoxyethane), alcohols (e.g., methanol, ethanol, propanol, isopropanol, butanol, isobutanol, sec-butanol and tert-butanol), esters (e.g., ethyl acetate), nitriles (e.g., acetonitrile), ketones (e.g., acetone, 2-butanone, 2-pentanone, 3-pentanone, 2-hexanone, 3-hexanone and methyl isobutyl ketone), pyridine, N,N-dimethylformamide (DMF), dimethylsulfoxide (DMSO), N-methylpyrrolidone (NMP), halogenated hydrocarbons (e.g., chloroform, dichloromethane, 1,2-dichloroethane and 1,1,2,2-tetrachloroethane), organic carboxylic acids (e.g., formic acid and acetic acid) and a mixed solvent thereof. The oxidant used includes, for example, hydrogen peroxide, organic peroxy acids such as performic acid, peracetic acid, pertrifluoroacetic acid, perbenzoic acid, m-chloroperbenzoic acid and monoperoxyphthalic acid, cumene hydroperoxide, N-halocarboxylic amides such as N-bromoacetamide, N-bromosuccinimide and N-chlorosuccinimide, tert-butyl hypochloride, manganese dioxide, and periodates such as orthoperiodic acid, sodium metaperiodate and potassium metaperiodate. In addition, this reaction may be performed in the presence of an acid catalyst. The acid catalyst includes, for example, organic acids such as formic acid, acetic acid and propionic acid, and mineral acids such as hydrochloric acid, sulfuric acid, nitric acid and perchloric acid. In addition, the metal catalyst includes, for example, vanadium oxide, vanadium oxide acetyl acetate, manganese oxide, molybdenum chloride and tungsten chloride. The amount of the oxidant used varies depending on the reaction conditions, but it is generally 1 to 100 mole equivalents, preferably 1 to 20 mole equivalents, based on the compound (VII). The reaction temperature is -50 to 200°C, preferably -30 to 150°C. The amount of the acid or metal catalyst used is 1/1000 to 100 mole equivalents, preferably 1/500 to 50 mole equivalents, based on the compound (VII).

### Process 4

The compound (IX) can be prepared by subjecting the compound (VIII) to deprotection reaction. The deprotection means include the hydrolysis and the hydrogenolysis. The hydrolysis is usually performed in a solvent in the presence of an acid or a base. The solvent includes, for example, ethers (e.g., dioxane, tetrahydrofuran (THF) and dimethoxyethane), alcohols (e.g., methanol, ethanol, propanol, isopropanol, butanol, isobutanol, sec-butanol and tert-butanol), ketones (e.g., acetone, 2-butanone, 2-pentanone, 3-pentanone, 2-hexanone, 3-hexanone and methyl isobutyl ketone), pyridine, N,N-dimethylformamide (DMF), dimethylsulfoxide (DMSO), N-methylpyrrolidone (NMP), water and a mixed solvent thereof. In the case where an acid is used, the acid includes organic acids such as formic acid, acetic acid and propionic acid, and mineral acids such as hydrochloric acid, sulfuric acid, nitric acid and perchloric acid. In the case where a base is used, the base includes an alkali metal salt (e.g., potassium hydroxide, sodium hydroxide, potassium carbonate, sodium carbonate, calcium carbonate, cesium carbonate, potassium hydrogencarbonate and sodium hydrogen carbonate). The amount of the acid or base used is 0.01 to 200 mole equivalents, preferably 0.1 to 100 mole equivalents, based on the compound (VIII). The reaction temperature is -20 to 200°C, preferably -10 to 100°C.

The hydrogenolysis reaction is usually performed in a solvent in the presence of a catalyst. The solvent includes, for example, aromatic hydrocarbons (e.g., benzene, toluene and xylene), ethers (e.g., dioxane, tetrahydrofuran (THF) and dimethoxyethane), esters (e.g., ethyl acetate, etc.), nitriles (e.g., acetonitrile, etc.), tertiary amines (e.g., pyridine, etc.), alcohols (e.g., methanol, ethanol, propanol, isopropanol, butanol, isobutanol, sec-butanol, tert-butanol, etc.), ketones (e.g., acetone, 2-butanone (MEK), methyl isobutyl ketone (MIBK), etc.), N,N-dimethylformamide (DMF), dimethylsulfoxide (DMSO), N-methylpyrrolidone (NMP), halogenated hydrocarbons (e.g., chloroform, dichloromethane, 1,2-dichloroethane, 1,1,2,2-tetrachloroethane, etc.), water and a mixed solvent thereof. As the catalyst, for example, palladiums such as palladium chloride, palladium black and palladium carbon, platinums such as platinum oxide, platinum black and platinum carbon, rhodiums such as rhodium carbon, Raney nickel and Raney cobalt are used. As the hydrogen source, hydrogen, formic acid, ammonium formate, isopropanol, etc. are used. The reaction temperature is -70 to 200°C, preferably 0 to 100°C. The reaction pressure is 0 to 10 MPa, preferably 0 to 5 MPa. The amount of the catalyst used is 1/10000 to 100 mole equivalents, preferably 1/1000 to 50 mole equivalents, based on the compound (VIII). For this hydrogenolysis reaction, an acid or a base may be added, if necessary. As the acid, organic acids such as formic acid, acetic acid and propionic acid, mineral acids such as hydrochloric acid, sulfuric acid, nitric acid and perchloric acid, etc. are used, and as the base, an alkali metal salt (e.g., potassium hydroxide, sodium hydroxide, potassium carbonate, sodium carbonate, potassium hydrogen carbonate, sodium hydrogen carbonate, etc.), amines (e.g., trimethylamine, triethylamine, diisopropyl ethylamine, N-methylmorpholine, 1,8-diazabicyclo[5.4.0]-7-undecene (DBU), 1,4-diazabicyclo[2,2,2]octane (DABCO), etc.), aromatic amines (e.g., N,N-dimethylaminopyridine, N,N-diethylamino pyridine, pyridine, 4-dimethylaminopyridine, picoline, quinoline, etc.), etc. are used.

### Process 5

Using the compound (IX) and the optically active acid represented by the formula (XIIa) or (XIIb), optical resolution is performed via a diastereomeric salt. In the process of preparing a diastereomeric salt, the reaction is performed in an appropriate solvent. The solvent includes, for example, aromatic hydrocarbons such as benzene, toluene and xylene, ethers such as dioxane, tetrahydrofuran (THF) and dimethoxyethane, alcohols such as methanol, ethanol, propanol, isopropanol, butanol, isobutanol, sec-butanol and tert-butanol, ethyl acetate, acetonitrile, ketones (e.g., acetone, 2-butanone, 2-pentanone, 3-pentanone, 2-hexanone, 3-hexanone, methyl isobutyl ketone, etc.), pyridine, N,N-dimethylformamide (DMF), dimethylsulfoxide (DMSO), N-methylpyrrolidone (NMP), water, chloroform, dichloromethane, 1,2-dichloroethane, 1,1,2,2-tetrachloroethane and a mixed solvent thereof. The acidic optical resolution agent includes, for example, tartaric acid and the derivative thereof [diacyl tartaric acid (diacetyl tartaric acid, dibenzoyl tartaric acid, di-p- toluoyl tartaric acid, di-1-naphthoyl tartaric acid, etc.), etc.], amino acid (pyroglutaric acid, aspartic acid and α-phenylglycine) and the derivative thereof [N-acylamino acid (N-acetylleucine, N-acetylvaline, N-(3,5-dinitro)benzoylphenylglycine, etc.), etc.] and (+) or (-) phosphoric acid such as a cyclic phosphate derivative (2,2'-(1,1'-binaphthyl)phosphoric acid and 4-phenyl-2-hydroxy-5,5-dimethyl-1,3,2-dioxaphosphorinan-2-oxide. The optical resolution agent used is preferably dibenzoyl tartaric acid, di-p-toluoyl tartaric acid, di-1-naphthoyl tartaric acid and N-(3,5-dinitro)benzoylphenylglycine, and the amount used thereof is 0.1 to 10 mole equivalents, preferably 0.5 to 5 mole equivalents, based on the compound (IX). The reaction temperature is -20 to 200°C, preferably -10 to 100°C. Process 6

The compound (X) can be prepared by reacting the compound (IV) with the compound (VI). For this reaction, the same reaction conditions as those in Process 2 can be employed.

### Process 7

The reaction for derivating the compound (II) from the compound (X) is performed by reacting the compound (X) with an oxidant in the presence of the optically active acid represented by the formula (XIIa) or the formula (XIIc). This reaction is performed in an appropriate solvent. The solvent includes, for example, aromatic hydrocarbons such as benzene, toluene and xylene, ethers such as dioxane, tetrahydrofuran (THF) and dimethoxyethane, alcohols such as methanol, ethanol, propanol, isopropanol, butanol, isobutanol, sec-butanol and tert-butanol, ethyl acetate, acetonitrile, ketones (e.g., acetone, 2-butanone, 2-pentanone, 3-pentanone, 2-hexanone, 3-hexanone, methyl isobutyl ketone, etc.), pyridine, N,N-dimethylformamide (DMF), dimethylsulfoxide (DMSO), N-methylpyrrolidone (NMP), water, chloroform, dichloromethane, 1,2-dichloroethane, 1,1,2,2-tetrachloroethane, organic carboxylic acids (e.g., formic acid, acetic acid, etc.) and a mixed solvent thereof. The oxidant used includes, for example, hydrogen peroxide, organic peroxy acids such as performic acid, peracetic acid, pertrifluoroacetic acid, perbenzoic acid, m-chloroperbenzoic acid and monoperoxyphthalic acid, cumene hydroperoxide, N-halocarboxylic acids such as N-bromoacetamide, N-bromosuccinimide and N-chlorosuccinimide, tert-butyl hypochloride, manganese dioxide, periodates such as orthoperiodic acid, sodium metaperiodate and potassium metaperiodate. In addition, this reaction may be performed in the presence of an acid catalyst. The acid catalyst includes, for example, a tartaric acid derivative [diacyl tartaric acid (dibenzoyl tartaric acid, di-p-toluoyl tartaric acid, di-1-naphthoyl tartaric acid, etc.), etc.], and (+) or (-) phosphoric acid such as a cyclic phosphate derivative (2,2'-(1,1'-binaphthyl)phosphoric acid and 4-phenyl-2-hydroxy-5,5-dimethyl-1,3,2-dioxaphosphorinan-2-oxide. Preferably, dibenzoyl tartaric acid, di-p-toluoyl tartaric acid, di-1-naphthoyl tartaric acid and 2,2'-(1,1'-binaphthyl)phosphoric acid are used. The amount of the optically active acid used is 0.5 to 10 mole equivalents, preferably 0.5 to 5 mole equivalents, based on 1 mole of the compound (X). The amount of the oxidant used is 1 to 100 mole equivalents, preferably 1 to 50 mole equivalents, based on 1 mole of the compound (X). The amount of the acid used is 0.1 to 10 mole equivalents, preferably 0.5 to 5 mole equivalents, based on 1 mole of the compound (X). The reaction temperature is -50 to 200°C, preferably -30 to 50°C.

### Process 8

The compound (IX) is prepared by oxidizing the compound (X). For this reaction, the same reaction conditions as those in Process 3 can be employed.

### Process 9

Metathesis reaction of the compound (XI) is performed in an appropriate solvent by contacting it with an acid or a base. The solvent includes, for example, aromatic hydrocarbons such as benzene, toluene and xylene, ethers such as dioxane, tetrahydrofuran (THF) and dimethoxyethane, alcohols such as methanol, ethanol, propanol, isopropanol, butanol, isobutanol, sec-butanol and tert-butanol, ethyl acetate, acetonitrile, ketones (e.g., acetone, 2-butanone, 2-pentanone, 3-pentanone, 2-hexanone, 3-hexanone, methyl isobutyl ketone, etc.), N,N-dimethylformamide (DMF), dimethylsulfoxide (DMSO), N-methylpyrrolidone (NMP), water, chloroform, dichloromethane, 1,2-dichloroethane, 1,1,2,2-tetrachloroethane and a mixed solvent thereof. As the acid, organic acids such as formic acid, acetic acid and propionic acid, and mineral acids such as hydrochloric acid, sulfuric acid, nitric acid and perchloric acid are used, and as the base, an alkali metal salt (e.g., potassium hydroxide, sodium hydroxide, potassium carbonate, sodium carbonate, potassium hydrogencarbonate, sodium hydrogen carbonate, etc.), amines (e.g., trimethylamine, triethylamine, diisopropyl ethylamine, N-methylmorpholine, 1,8-diazabicyclo[5.4.0]-7-undecene (DBU), 1,4-diazabicyclo[2,2,2]octane (DABCO), etc.) and aromatic amines (e.g., N,N-dimethylaminopyridine, N,N-diethylamino pyridine, pyridine, 4-dimethylaminopyridine, picoline, quinoline, etc.) are used. The reaction temperature is -30 to 150°C, preferably -10 to 100°C. The amount of the acid used or base used is 1 to 200 mole equivalents, preferably 1 to 100 mole equivalents, based on 1 mole of the compound (XI).

### Process 10

The compound (I) can be prepared by reacting the compound (II) with the compound (III) or a reactive derivative thereof. This reaction is usually performed in a solvent. Any solvent can be used if it does not inhibit the reaction, but it includes for example, aromatic hydrocarbons such as benzene, toluene and xylene, ethers such as dioxane, tetrahydrofuran (THF) and dimethoxyethane, ethyl acetate, acetonitrile, ketones (e.g., acetone, 2-butanone, 2-pentanone, 3-pentanone, 2-hexanone, 3-hexanone, methyl isobutyl ketone, etc.), pyridine, N,N-dimethylformamide (DMF), N,N-dimethylacetoamide (DMA), dimethylsulfoxide (DMSO), N-methylpyrrolidone (NMP), chloroform, dichloromethane, 1,2-dichloroethane, 1,1,2,2-tetrachloroethane, and a mixed solvent thereof. The reactive derivative means the reactive derivative in the carboxyl group of the compound represented by the formula (III). The reactive derivative includes, for example, those which are well-known in the field of peptide such as acid chloride, acid bromide, mixed acid anhydride and active ester. In addition, in the case where the reactive derivative is used, the reaction may be performed in the presence of a base. The base includes an alkali metal salt (e.g., potassium hydroxide, sodium hydroxide, potassium carbonate, sodium carbonate, potassium hydrogen carbonate and sodium hydrogen carbonate), amines (e.g., trimethylamine, triethylamine, diisopropyl ethylamine, N-methylmorpholine, 1,8-diazabicyclo[5.4.0]-7-undecene (DBU), 1,4-diazabicyclo[2,2,2]octane (DABCO), etc.) and aromatic amines (e.g., N,N-dimethylaminopyridine, N,N-diethylamino pyridine, pyridine, 4-dimethylaminopyridine, picoline, quinoline, etc.). The amount of the base used is preferably about 1 to 5 mole equivalents, based on the compound (III) or a reactive derivative thereof.

In addition, the amount of the compound (III) or a reactive derivative used is preferably about 1 to 5 mole equivalents, based on Compound (II). The reaction temperature is usually -20 to 150°C, preferably -10 to 100°C. The reaction time is about 1 to 100 hours.

The compound (III) which is used as a starting material in the reaction can be prepared according to the method as described in WO01-17947 (corresponding to JP 2001-151741 A).

### Examples and Reference Examples

The present invention is hereinafter described in more detail by means of the following Examples and Reference Examples, but the present invention is not limited thereto.

### Reference Example 1

### 1-Isobutylpiperidin-2-one

To a suspension of potassium hydroxide (482 g) in toluene (700 ml) was added dropwise a solution of 2-piperidone (181 g) in toluene (100 ml) at 50°C or lower. Then, to the reaction mixture was added tetrabutylammonium bromide (5.88 g) and further added dropwise a solution of isobutyl bromide (500 g) in toluene (100 ml). The mixture was stirred at 50 to 60°C for 3 hours and then allowed to cool to room temperature. Water (500 ml) was added to the reaction mixture and the resulting solution was neutralized with concentrated hydrochloric acid (460 ml) under ice-cooling (pH 8) and separated into layers. The aqueous layer was reextracted with toluene (450 ml), the organic layers were combined and washed with saturated brine, and then the solvent was distilled off. The residue was distilled under reduced pressure and a fraction of b.p. 98°C was collected at 8 mmHg to obtain 23.5 g (yield: 83.1%) of the title compound as a colorless oily matter.
¹H-NMR (300MHz, CDCl₃, δ): 0.86 (6H, d, J = 6.7Hz), 1.73-1.78 (4H, m), 1.85-2.05 (1H, m), 2.34-2.38 (2H, m), 3.16 (2H, d, J = 7.7Hz), 3.20-3.25 (2H, m).

### Reference Example 2

### 8-Bromo-1-isobutyl-1,2,3,4-tetrahydro-1-benzazocinee-5-carboxylic acid

To 1-isobutylpiperidin-2-one (10 g) were added methanesulfonic acid (16.7 ml) and water (35 ml), and the mixture was stirred under reflux for 24 hours to form 5-isobutylaminopentanoic acid. The reaction solution was cooled to 50°C and neutralized with sodium carbonate (13.7 g). The neutralized solution was slowly added dropwise at 110 to 115°C to a mixture of 2-fluoro-5-bromobenzaldehyde (8.2 g), sodium carbonate (11.1 g), and DMSO (65 ml), and the mixture was stirred with heating under reflux for 4 hours. The mixture was cooled to 50°C, water (30 ml) and toluene (40 ml) were added thereto, and the resulting solution was adjusted to pH 3.5 with 6 N hydrochloric acid. The toluene layer was separated and the aqueous layer was reextracted with toluene (40 ml). The organic layers were combined and washed with 10% brine and water. The solvent was distilled off to obtain 5-[(4-bromo-2-formylphenyl)(isobutyl)amino]pentanoic acid of a brown oily matter. This compound was dissolved in N,N-dimethylformamide (24.6 ml), and to the solution was added potassium carbonate (56.7 g) and then added dropwise methyl iodide (3 ml). The mixture was stirred at room temperature for 4 hours, thereto was added dimethyl carbonate (49.2 ml) and then added dropwise a solution of 28% sodium methoxide in methanol (15.6 g), and the mixture was stirred at 60°C for 1.5 hours. Water was added to the reaction solution and the mixture was extracted with toluene. The organic layer was washed with 10% brine and then water. The solvent was distilled off to obtain methyl 8-bromo-1-isobutyl-1,2,3,4-tetrahydro-1-benzazocinee-5-carboxylate of a brown oily matter.

To a solution of methyl 8-bromo-1-isobutyl-1,2,3,4-tetrahydro-1-benzazocinee-5-carboxylate in tetrahydrofuran - methanol (2 : 1, 45 ml) was added an aqueous 2 N sodium hydroxide solution (40 ml) and the reaction mixture was stirred at 60°C for 2.5 hours. The mixture was allowed to cool to room temperature and adjusted to pH 3.5 with concentrated hydrochloric acid, and the mixture was extracted with toluene. The organic layer was washed with water, activated carbon (0.8 g) was added thereto, and the mixture was stirred for 10 minutes. The activated carbon was filtered off and the solvent was then distilled off. The residue was dissolved in methanol (24 ml) at 60°C and to the solution was added dropwise water (6 ml). The mixture was stirred at room temperature for 1 hour and crystallized. The crystals were collected by filtration, washed with methanol - water (5 : 2), and then dissolved in methanol (24 ml) with heating under reflux. To the solution was added dropwise water (4.8 ml) at the same temperature, and the mixture was stirred at room temperature for 1 hour and crystallized. The crystals were collected by filtration, washed with methanol - water (5 : 1), and dried at 40°C under reduced pressure to obtain 6.2 g (yield: 45%) of the title compound as yellow powder.
¹H-NMR (300MHz, CDCl₃, δ) : 0.97 (6H, d, J = 6.7Hz), 1.43-1.47 (2H, m), 2.05-2.25 (1H, m), 2.45-2.55 (2H, m), 3.00 (2H, d, J = 7.3Hz), 3.40-3.50 (2H, m), 6.63 (1H, d, J =8.9Hz), 7.15-7.30 (2H, m), 7.83 (1H, s).

| Elemental analysis value: in terms of C₁₆H₂₀NO₂Br | | | | |
|---|---|---|---|---|
| Calcd. value | C, 56.82; | H, 5.96; | N, 4.14; | Br, 23.62 |
| Found value | C, 56.63; | H, 5.88; | N, 3.92; | Br, 23.41 |

IR (KBr, cm⁻¹): 1664, 1604, 1494
Melting point: 153.3 - 154.0°C

### Reference Example 3

### 8-[9-(2-Butoxyethoxy)phenyl]-1-isobutyl-1,2,3,4-tetrahydro-1-benzazocine-5-carboxylic acid

Under an argon atmosphere, to a suspension of magnesium (1.3 g) in tetrahydrofuran (100 ml) was slowly added dropwise one third of a solution of 1-bromo-4-(2-butoxyethoxy)benzene (14.2 g) in THF (30 ml) with heating under reflux. After confirming that the reaction was initiated, to the solution were added dropwise the other two thirds thereof, and the mixture was stirred for 1 hour under reflux. To the mixture was added dropwise a solution of trimethyl borate (5.7 ml) in tetrahydrofuran (30 ml) while keeping the temperature at -10°C or lower and the reaction mixture was stirred for 1 hour. The reaction mixture was warmed to room temperature, thereto were added palladium acetate (20 mg) and triphenylphosphine (93 mg), and the mixture was stirred for 30 minutes. To the mixture were added 8-bromo-1-isobutyl-1,2,3,4-tetrahydro-1-benzazocine-5-carboxylic acid (10 g), tripotassium phosphate (39.3 g) and distilled water (80 ml), and the resulting mixture was stirred for 3 hours with heating under reflux. After allowing to cool the mixture, water (100 ml) was added thereto, and the resulting mixture was adjusted to pH 3 with 6 N hydrochloric acid under ice-cooling and separated into layers. The aqueous layer was further extracted with ethyl acetate. The organic layers were combined and washed with saturated brine, activated carbon (1 g) and tributylphosphine (1 ml) were added thereto, and the mixture was stirred at room temperature for 20 minutes. The activated carbon was filtered off and the solvent was then distilled off. To the residue was added isopropyl ether (50 ml) and dissolved therein at 60°C. The mixture was stirred at room temperature for 2 hours and then stirred for 1 hour under ice-cooling, and the crystals were collected by filtration (12.6 g). The crystals were dissolved in isopropanol (55 ml) at 60°C, to the solution was added activated carbon (1 g), and the mixture was stirred for 20 minutes. The activated carbon was filtered off while the mixture was hot, and the mixture was stirred at 60°C for 30 minutes, stirred for 1 hour at room temperature and then stirred for 1 hour under ice-cooling. The crystals were collected by filtration, washed with cold isopropanol (10 ml) and dried at 40°C under reduced pressure to obtain 5.30 g (yield: 89%) of the title compound as yellow powder.
¹H-NMR (300MHz, CDCl₃, δ): 0.94 (3H, t, J = 7.3Hz), 1.00 (6H, d, J = 6.6Hz), 1.35-1.65 (6H, m), 2.10-2.30 (1H, m), 2.55-2.60 (2H, m), 3.08 (2H, d, J = 7.2Hz), 3.45-3.75 (4H, m), 3.80-3.85 (2H, m), 4.15-4.20 (2H, m), 6.83 (1H, d, J = 8.8Hz), 6.96 (2H, d, J = 8.7Hz), 7.30-7.40 (2H, m), 7.43 (2H, d, J = 8.7Hz), 8.01 (1H, s).

| Elemental analysis value: in terms of C₂₈H₃₇NO₄ | | | |
|---|---|---|---|
| Calcd. value | C, 74.47; | H, 8.26; | N, 3.10 |
| Found value | C, 74.53; | H, 8.51; | N, 3.03 |

IR (KBr, cm⁻¹): 1666, 1604, 1494
Melting point: 119.6 - 120.1°C

### Reference Example 4

### 8-[4-(2-Butoxyethoxy)phenyl)-1-isobutyl-1,2,3,4-tetrahydro-1-benzazocine-5-carboxylic acid

Under an argon atmosphere, to a suspension of magnesium (650 mg) in tetrahydrofuran (50 ml) was added trimethylsilyl chloride (0.07 ml) and the reaction mixture was stirred at room temperature for 10 minutes. To the mixture was added dropwise one third of a solution of 1-bromo-4-(2-butoxyethoxy)benzene (7.08 g) in tetrahydrofuran (15 ml). The mixture was slowly warmed (23°C → 32°C). After confirming that the reaction was initiated by coloration in about 30 minutes, to the reaction solution were added dropwise the other two thirds thereof and after completion of addition, the mixture was stirred at 45 to 50°C for 0.5 hours. The mixture was allowed to cool to room temperature, thereto was added dropwise a solution of trimethyl borate (2.9 ml) in tetrahydrofuran (15 ml) while keeping the temperature at -10°C or lower, and the reaction mixture was stirred for 1 hour. The reaction mixture was warmed to room temperature, thereto were added palladium acetate (10 mg) and triphenylphosphine (47 mg), and the mixture was stirred for 30 minutes. To the mixture were added 8-bromo-1-isobutyl-1,2,3,4-tetrahydro-1-benzazocine-5-carboxylic acid (5 g), tripotassium phosphate (19.63 g) and distilled water (40 ml), and the resulting mixture was stirred for 2 hours with heating under reflux. The mixture was allowed to cool, water (50 ml) was added thereto, and the resulting mixture was adjusted to pH 3 with 6 N hydrochloric acid and separated into layers. The aqueous layer was extracted with ethyl acetate (50 ml). The organic layers were combined and washed with saturated brine, activated carbon (0.5 g) and tributylphosphine (0.5 ml) were added thereto, and the mixture was stirred at room temperature for 20 minutes. The activated carbon was filtered off and the solvent was then distilled off. To the residue was added isopropyl ether (25 ml), and the mixture was heated at 60°C and slowly returned to room temperature, and stirred at room temperature for two hours and then stirred for 1 hour under ice-cooling. The crystals were collected by filtration (6.0 g) and washed with cold isopropyl ether (10 ml). The crystals were dissolved in isopropanol (60 ml) at 60°C, to the solution was added activated carbon (0.5 g), and the mixture was stirred for 20 minutes. The activated carbon was filtered off and the solvent was then distilled off. To the residue was added isopropanol (30 ml), followed by dissolving therein at 60°C. The mixture was stirred at room temperature for 3 hours and then stirred for 1 hour under ice-cooling. The crystals were collected by filtration, washed with cold isopropanol (10 ml) and dried at 40°C under reduced pressure to obtain 5.38 g (yield: 80.5%) of the title compound as yellow powder.

### Reference Example 5

### 1-Isobutylpyrrolidin-2-one

To a solution of 2-pyrrolidone (60 g) in tetrahydrofuran (300 ml) were added potassium hydroxide (59.3 g) and tetrabutylammonium bromide (2.5 g) and then added dropwise isobutyl bromide (144.9 g) at 50°C or lower. The resulting mixture was stirred at 50 to 60°C for 3 hours and allowed to cool to room temperature. Insoluble materials were filtered off and washed with toluene (300 ml). The filtrate was washed with saturated brine (150 ml), and then the solvent was distilled off. The residue was distilled under reduced pressure and a fraction of b.p. 97 to 102°C was collected at 3 mmHg to obtain 73 g (yield: 73%) of the title compound as a colorless oily matter. Reference Example 6

### 7-Bromo-1-isobutyl-2,3-dihydro-1H-2-benzazepine-4-carboxylic acid

To 1-isobutylpyrrolidin-2-one (28 g) were added water (66 ml) and methanesulfonic acid (26 ml) and the resulting mixture was stirred for 24 hours under reflux. After allowing to cool the mixture, thereto were added water (30 ml) and sodium carbonate (84.8 g) (vigorously foamed), and the mixture was stirred for 1 hour. To the reaction solution was added dimethylsulfoxide (30 ml) and added dropwise a solution of 5-bromo-2-fluorobenzaldehyde (12.2 g) in dimethylsulfoxide (66 ml). After completion of addition, the mixture was heated under reflux for 9 hours. After allowing to cool, the mixture was adjusted to pH 3 with 6 N hydrochloric acid and extracted with ethyl acetate. The organic layer was washed with 20% brine and the product was extracted with an aqueous 1 N sodium hydroxide solution (100 ml). The aqueous layer was adjusted to pH 3 with 6 N hydrochloric acid and extracted with ethyl acetate. The solvent was distilled off to obtain 4-[(4-bromo-2-formylphenyl)(isobutyl)amino]butanoic acid as a brown oily matter. To a solution of 4-[(4-bromo-2-formylphenyl)(isobutyl)amino]butanoic acid in N,N-dimethylformamide (50 ml) was added potassium carbonate (9.12 g) and added dropwise a solution of methyl iodide (10.22 g) in N,N-dimethylformamide (10 ml). After stirring for 1 hour at room temperature, to the resulting solution was added dimethyl carbonate (120 ml) and added dropwise 28% sodium methoxide (27.8 g), and the mixture was stirred at 60°C for 1 hour. The mixture was adjusted to pH 3 with 2 N hydrochloric acid (100 ml) under ice-cooling. Then, the mixture was adjusted to pH 6.5 with an aqueous 6 N sodium hydroxide solution and the solvent was distilled off. The residue was extracted with toluene. The toluene layer was sequentially washed with an aqueous 1 N sodium hydroxide solution, 2% brine and water. The solvent was distilled off to obtain methyl 7-bromo-1-isobutyl-2,3-dihydro-1H-1-benzazepine-4-carboxylate as a brown oily matter.

To a solution of the oily matter in tetrahydrofuran - methanol (1 : 1, 190 ml) was added an aqueous 1 N sodium hydroxide solution (120 ml) and the reaction mixture was stirred at 50°C for 1.5 hours. After distilling off about half of the solvent, the residue was washed with toluene. The aqueous layer was adjusted to pH 3 with 6 N hydrochloric acid and extracted with ethyl acetate. The ethyl acetate layer was washed with 2% brine and the solvent was then distilled off to obtain a brown oily matter. The oily matter was dissolved in methanol (120 ml), thereto was added activated carbon (1.2 g), and the mixture was stirred 60°C for 20 minutes. The activated carbon was filtered off and the solvent was then distilled off. The residue was dissolved in methanol (45 ml) at 60°C, to the solution was added water (9 ml), and the crystals were precipitated. The mixture was stirred for 8 hours at room temperature and then stirred for 1 hour under ice-cooling. The crystals were collected by filtration, washed with cold methanol - water (1 : 1, 30 ml) and dried at 40°C under reduced pressure to obtain 10.13 g (yield: 52.1%) of the title compound.
¹H-NMR (300MHz, CDCl₃, δ): 0.91 (6H, d, J = 6.6Hz), 1.90-2.25 (1H, m), 2.75-2.85 (2H, m), 3.12 (2H, d, J = 7.4Hz), 3.20-3.30 (2H, m), 6.72 (1H, d, J = 9.0Hz), 7.20-7.30 (1H, m), 7.44 (1H, s), 7.70 (1H, s).

| Elemental analysis value: in terms of C₁₅H₁₈NO₂Br | | | | |
|---|---|---|---|---|
| Calcd. value | C, 55.57; | H, 5.60; | N, 4.32; | Br, 24.65 |
| Found value | C, 55.52; | H, 5.59; | N, 4.13; | Br, 24.39 |

IR (KBr, cm⁻¹): 1677, 1614, 1490
Melting point: 144.8 - 145.5°C

### Reference Example 7

### 7-[4-(2-Butoxyethoxy)phenyl]-1-isobutyl-2,3-dihydro-1H-1-benzazepine-4-carboxylic acid

Under an argon atmosphere, a suspension of magnesium (2.25 g) in tetrahydrofuran (140 ml) was heated under reflux and to the mixture was slowly added dropwise one third of a solution of 1-bromo-4-(2-butoxyethoxy)benzene (24.6 g) in tetrahydrofuran (40 ml). After confirming that the reaction was initiated (the reaction solution was clouded), to the solution were added dropwise the other two thirds thereof, and the mixture was stirred for 1 hour under reflux. After allowing cool the reaction solution to room temperature, thereto was added dropwise a solution of trimethyl borate (10 ml) in tetrahydrofuran (40 ml) while keeping the temperature at -10°C or lower and the reaction solution was stirred for 1 hour. The reaction solution was warmed to room temperature, thereto were added palladium acetate (27 mg) and triphenylphosphine (126 mg), and the mixture was stirred for 30 minutes. To the mixture were added 7-bromo-1-isobutyl-2,3-dihydro-1H-1-benzazepine-4-carboxylic acid (19.45 g), tripotassium phosphate (79.6 g) and distilled water (100 ml), and the resulting solution was stirred for 2 hours with heating under reflux. After allowing to cool the mixture, water (150 ml) was added thereto, and the resulting solution was adjusted to pH 3 with 6 N hydrochloric acid (150 ml) and separated into layers. The aqueous layer was extracted with toluene (140 ml). The organic layer was sequentially washed with an aqueous 1 N sodium hydroxide solution, 1 N hydrochloric acid and 20% brine. Activated carbon (2 g) and tributylphosphine (2 ml) were added to the organic layer and the mixture was stirred for 20 minutes at room temperature. The activated carbon was filtered off and the solvent was then distilled off. To the residue was added isopropyl ether (80 ml), followed by heating. The mixture was stirred for 1 hour at room temperature and stirred for 1 hour under ice-cooling, and the crystals were collected by filtration. The crystals were washed with isopropyl ether (30 ml) and dried at 40°C under reduced pressure to obtain 23.72 g (yield: 90.3%) of yellow crystals. The crystals (2 g) were dissolved in isopropanol (10 ml) with heating under reflux, to the resulting solution was added tributylphosphine (0.2 ml), and the mixture was stirred for 2 hours at room temperature and stirred for 1 hour under ice-cooling. The crystals were collected by filtration, washed with cold isopropanol (5 ml) and dried at 40°C under reduced pressure to obtain 1.92 g (yield: 96.0%) of the pure title compound as yellow crystals.
¹H-NMR (300MHz, CDCl₃, δ): 0.90-1.10 (9H, m), 1.35-1.50 (2H, m), 1.55-1.70 (2H, m), 2.00-2.15 (1H, m), 2.85-2.90 (2H, m), 3.20 (2H, d, J = 7.3Hz), 3.30-3.35 (2H, m), 3.58 (2H, d, J = 6.7Hz), 3.80-3.85 (2H, m), 4.15-4.20 (2H, m), 6.92 (1H, d, J = 8.8Hz), 7.01 (2H, d, J = 8.8Hz), 7.40-7.55 (4H, m), 7.91 (1H, s).

| Elemental analysis value: in terms of C₂₇H₃₅NO₄ | | | |
|---|---|---|---|
| Calcd. value | C, 74.11; | H, 8.06; | N, 3.20 |
| Found value | C, 74.18; | H, 8.33; | N, 2.95 |

IR (KBr, cm⁻¹): 1668, 1608, 1500
Melting point: 126.4 - 127.0°C

### Reference Example 8

### 1-Propylpiperidin-2-one

To toluene (750 ml) were added potassium hydroxide (254 g) and then added tetrabutylammonium bromide (4.87 g) and to the resulting mixture was slowly added dropwise a solution of 2-piperidone (150 g) in toluene (150 ml) at 50 to 60°C. The mixture was stirred at the same temperature for 1 hour. To the mixture was added dropwise propyl bromide (371 g) at the same temperature. The mixture was stirred at the same temperature for 1 hour and thereto was added water (450 ml) while keeping the temperature at 30°C or lower. The mixture was adjusted to pH 7.2 by adding dropwise 48% hydrobromic acid and then the organic layer was separated. The aqueous layer was extracted with toluene (450 ml). The organic layers were combined and concentrated under reduced pressure. The residue was distilled under reduced pressure and a fraction of b.p. 91 to 93°C was collected at 6 mmHg to obtain 169.3 g (yield: 79%) of the title compound as a colorless oily matter.
¹H-NMR (CDCl₃, δ, 300MHz): 0.88 (3H, t, J = 7.4Hz), 1.48-1.61 (2H, m), 1.74-2.00 (4H, m), 2.33-2.38 (2H, m), 3.22-3.33 (4H, m).

### Reference Example 9

### 8-Bromo-1-propyl-1,2,3,4-tetrahydro-1-benzazocine-5-carboxylic acid

1-Propylpiperidin-2-one (55.6 g) was added to 4 N sodium hydroxide (197 ml) and the resulting mixture was refluxed for 4 hours. The mixture was cooled to room temperature and adjusted to pH 6.1 with adding concentrated hydrochloric acid. To the mixture were added sodium carbonate (83.5 g), water (255 ml), dimethylsulfoxide (520 ml), and 5-bromo-2-fluorobenzaldehyde (40 g). After refluxing for 5 hours, the mixture was cooled to 50°C and adjusted to pH 3.1 with 6 N hydrochloric acid. The mixture was cooled to room temperature and extracted with ethyl acetate. The organic layers were combined, washed with 5% brine and the product was extracted with an aqueous 5% sodium carbonate solution (440 ml). The aqueous layer was adjusted to pH 3.3 with 6 N hydrochloric acid and extracted with a mixed solution of toluene (400 ml) and tetrahydrofuran (150 ml). The organic layer was sequentially washed with 5% brine and water, and the solvent was distilled off to obtain 5-[(4-bromo-2-formylphenyl)(propyl)amino]valeric acid as a brown oily matter. The obtained 5-[(4-bromo-2-formylphenyl)(propyl)amino]valeric acid was dissolved in N,N-dimethylformamide (120 ml), thereto was added potassium carbonate (30 g) and further added methyl iodide (33.6 g), and the resulting mixture was stirred at room temperature for 2 hours. To the mixture was added dimethyl carbonate (240 ml) and then added dropwise a solution of 28% sodium methoxide in methanol (91.2 g), and the mixture was stirred at 60°C for 1 hour. The mixture was cooled to room temperature, thereto was added water (400 ml) and the mixture was extracted with toluene. The organic layer was washed with 5% brine and concentrated under reduced pressure to obtain methyl 8-bromo-1-propyl-1,2,3,4-tetrahydro-1-benzazocine-5-carboxylate as an oily matter. The obtained methyl 8-bromo-1-propyl-1,2,3,4-tetrahydro-1-benzazocine-5-carboxylate was dissolved in a mixed solution of tetrahydrofuran (100 ml) and methanol (100 ml). To the solution was added 2 N sodium hydroxide (197 ml) and the reaction mixture was refluxed for 1 hour. The mixture was cooled to room temperature and adjusted to pH 3.2 with adding 6 N hydrochloric acid. The resulting mixture was extracted with ethyl acetate and the extract was washed with 5% brine. To the extract was added activated carbon (2 g), the resulting mixture was stirred for 30 minutes and then filtered, and the mixture was washed with ethyl acetate (80 ml). The filtrates were combined and concentrated under reduced pressure, and to the obtained crystals were added methanol (90 ml). The resulting mixture was refluxed for 30 minutes, and stirred at room temperature for 1 hour and stirred for 2 hours under ice-cooling. The obtained crystals were collected by filtration to obtain 29.76 g of the title compound (yield: 47%)
¹H-NMR (CDCl₃, δ, 300MHz): 0.96 (3H, t, J = 7.4Hz), 1.40-1.49 (2H, m), 1.59-1.71 (2H, m), 2.53 (2H, t, J = 6.0Hz), 3.12 (2H, t, J = 7.9Hz), 3.44 (2H, t, J = 5.3Hz), 6.56-6.60 (1H, m), 7.19-7.23 (2H, m), 7.81 (1H, s).

### Reference Example 10

### 8-[4-(2-Butoxyethoxy)phenyl]-1-propyl-1,2,3,4-tetrahydro-1-benzazocine-5-carboxylic acid

Under an argon atmosphere, magnesium (675 mg) was added to tetrahydrofuran (50 ml). To the resulting suspension was slowly added dropwise a solution of 1-bromo-4-(2-butoxyethoxy)benzene (7.37 g) in tetrahydrofuran (15 ml) under reflux and the mixture was refluxed for 1 hour. To the mixture was added dropwise a solution of trimethyl borate (2.79 g) in tetrahydrofuran (15 ml) at -15 to -10°C and the mixture was stirred at the same temperature for 30 minutes. The mixture was warmed to room temperature, thereto were added palladium acetate (13 mg) and triphenylphosphine (63 mg), and the mixture was stirred at room temperature for 30 minutes. To the mixture were sequentially added 8-bromo-1-propyl-1,2,3,4-tetrahydro-1-benzazocine-5-carboxylic acid (5.0 g), potassium phosphate (20.5 g) and distilled water (40 ml), and the resulting mixture was refluxed with stirring for 2.5 hours. To the reaction solution were added palladium acetate (13 mg) and triphenylphosphine (63 mg), and then the mixture was refluxed for 50 minutes. The mixture was cooled to room temperature and water (40 ml) was added thereto. The resulting solution was adjusted to pH 3.3 with 6 N hydrochloric acid under ice-cooling and the organic layer was separated. The aqueous layer was extracted with toluene. The organic layers were combined and washed with water and 1 N sodium hydroxide. To the organic layer was added 0.3 N hydrochloric acid (120 ml) and further added ethyl acetate, and then insoluble materials was filtered and the filtrate was separated. The organic layer was washed with water, thereto were added tributylphosphine (0.5 ml) and then activated carbon (250 mg), and the mixture was stirred at room temperature for 30 minutes. The mixture was filtered, washed with ethyl acetate and concentrated under reduced pressure. To the obtained crystals were added ethyl acetate (25 ml) and the mixture was stirred under reflux. Then, the mixture was stirred for 1 hour under ice-cooling. The obtained crystals were filtered and washed with the ice-cooled ethyl acetate (10 ml) to obtain 5.63 g (yield: 83%) of the title compound as reddish yellow crystals.
¹H-NMR (CDCl₃, δ, 300MHz): 0.90-1.01 (6H, m), 1.35-1.72 (8H, m), 2.57 (2H, t, J = 5.3Hz), 3.19 (2H, t, J = 7.7Hz), 3.48-3.57 (4H, m), 3.80 (2H, t, J = 4.9Hz), 4.15 (2H, t, J = 4.9Hz), 6.76 (1H, d, J = 8.9Hz), 6.95 (2H, d, J = 8.7Hz), 7.32-7.45 (4H, m), 8.00 (1H, s).

### Reference Example 11

### 5-(Chloromethyl)-1-propyl-1H-imidazole monohydrochloride

To a mixture of 1-butanol (900 ml) and acetic acid (120 ml) was added dropwise n-propylamine (103 ml) and further added dropwise methanesulfonic acid (81 ml) at 30°C or lower. The reaction mixture was returned to room temperature, thereto were added 1,3-dihydroxyacetone dimer (90.1 g) and potassium thiocyanate (145.8 g), and the resulting mixture was stirred at the same temperature for 24 hours. To the mixture was added water (250 ml), the reaction mixture was stirred at room temperature 2 hours, and then the precipitated crystals were collected by filtration. The obtained crystals were washed with water (100 ml) to obtain (2-mercapto-1-propyl-1H-imidazol-5-yl)methanol.

The solution of sodium nitrite (1.7 g) in water (5 ml) was added dropwise to 5 M nitric acid (600 ml) at 20°C or lower. To the solution was first added dropwise a solution of all of the above-obtained (2-mercapto-1-propyl-1H-imidazol-5-yl)methanol in aqueous 3.5 N sodium hydroxide (215 ml) at 20°C or lower and then the mixture was stirred at room temperature for 2 hours. Further, to the reaction mixture was added dropwise at 20°C or lower an aqueous 6 N sodium hydroxide solution (370 ml) (pH = about 6.5) and then added dropwise an aqueous 2 M sodium carbonate solution (370 ml) (pH = about 9) at 20°C or lower. The mixture was extracted with ethyl acetate - isopropanol (2 : 1, 450 ml) and again, extracted twice with ethyl acetate - isopropanol (3 : 1, 400 ml). The organic layers were combined, washed with 10% brine, and dried over anhydrous magnesium sulfate. The solvent was distilled off to obtain (1-propyl-1H-imidazol-5-yl)methanol as an oily matter.

To all of the above (1-propyl-1H-imidazol-5-yl)methanol was added DMF (375 ml)and the remaining ethyl acetate was distilled off under reduced pressure. Then, toluene (100 ml) was added thereto and the mixture was distilled off under reduced pressure. After carrying out this operation twice, to the residue was added dropwise thionyl chloride (40.1 ml) at 30°C or lower. The mixture was returned to room temperature and stirred for two hours. To the reaction mixture was added toluene (700 ml) and the resulting mixture was stirred at room temperature for 1 hour. The precipitated crystals were collected by filtration, washed with toluene (100 ml), and dried at 40°C under reduced pressure to obtain 63.7 g of the title compound as white crystals.
¹H-NMR (300MHz, CDCl₃, δ): 0.88 (3H, t, J = 7.35Hz), 1.80-2.00 (2H, m), 4.21 (2H, t, J = 7.35Hz), 5.05 (2H, s), 7.83 (1H, s), 9.33 (1H, s).

| Elemental analysis value: in terms of C₇H₁₁ClN₂·HCl | | | |
|---|---|---|---|
| Calcd. value | C, 43.10; | H, 6.20; | N, 14.36 |
| Found value | C, 43.12; | H, 5.95; | N, 14.28 |

IR (KBr, cm⁻¹): 1600
Melting point: 164.8 - 166.4°C

### Example 1

### 2,2,2-Trifluoro-N-(4-{[(1-propyl-1H-imidazol-5-yl)methyl]thio}phenyl)acetamide

Triethylamine (27.9 ml) was added dropwise to a solution of 4-aminobenzenethiol (12.5 g) in tetrahydrofuran (180 ml) at 0 to 10°C. Subsequently, to the reaction mixture was added dropwise trifluoroacetic anhydride (28.2 ml) at 0 to 10°C and the reaction mixture was stirred at the same temperature for 0.5 hours. To the reaction solution was added water (30 ml) and the mixture was stirred at room temperature for 0.5 hours. To the mixture was added 20 w/w% brine (30 ml) and the organic layer was separated. The aqueous layer was extracted with ethyl acetate (180 ml). The organic layers were combined and water (30 ml) was added thereto. The resulting solution was adjusted to about pH 9 with adding sodium hydrogen carbonate. After separation into layers, the organic layer was washed with water (30 ml) and concentrated. To the precipitated crystals were added n-hexane (120 ml) and the resulting solution was stirred at room temperature for 17 hours. The crystals were collected by filtration and washed with n-hexane (20 ml). The crystals were dried under reduced pressure to obtain 26.1 g of 2,2,2-trifluoro-N-(4-mercaptophenyl)acetamide as white crystals.
¹H-NMR (CDCl₃, 300MHz) δ: 3.45 (1H, s), 7.18 (2H, d, J = 9.4Hz), 7.51 (2H, d, J = 9.4Hz), 9.97 (1H, brs)

Under a nitrogen atmosphere, to a solution of the above obtained 2,2,2-trifluoro-N-(4-mercaptophenyl)acetamide (24.8 g) in methanol (99 ml) was added dropwise triethylamine (29.0 ml) at 0 to 10°C under ice-cooling. Subsequently, to the reaction mixture was added dropwise at 0 to 20°C a solution of 5-(chloromethyl)-1-propyl-1H-imidazole hydrochloride (20.4 g) in distilled water (21 ml) and the resulting solution was stirred at 20 to 30°C for 0.5 hours. Ethyl acetate (200 ml) was added to the reaction solution and the organic layer was separated. Subsequently, after washing with an aqueous 7 w/w% sodium hydrogen carbonate (50 ml) and water (50 ml), the organic layer was concentrated. To the precipitated crystals were added diisopropyl ether (250 ml) and the resulting mixture was stirred for 0.5 hours with heating under reflux, and then allowed to cool to room temperature and stirred for 3 hours. The precipitated crystals were collected by filtration and washed with diisopropyl ether (20 ml). The crystals were dried under reduced pressure to obtain 23.8 g (yield: 73%, calculated from the amount of 4-aminobenzenethiol) of the title compound as white crystals. Melting point: 82 - 84°C

| Elemental analysis value: in terms of C₁₅H₁₆N₃OSF₃·0.5 H₂O | | | |
|---|---|---|---|
| Calcd. value | C, 51.13; | H, 4.86; | N, 11.92 |
| Found value | C, 51.41; | H, 4.55; | N, 11.75 |

¹H-NMR (CDCl₃, 300MHz) δ: 0.99 (3H, t, J = 7.4Hz), 1. 67 (1H, brs), 1.82-1.94 (2H, m), 3.77 (2H, t, J = 6.6Hz), 3.99 (2H, s), 6.67 (1H, s), 6.96-7.31 (2H, m), 7.47 (1H, m), 7.51-7.59 (2H, m)
IR (KBr, cm⁻¹) : 1704, 1504, 1247, 1195, 1160, 1145, 1108

### Example 2

### 2,2,2-Trifluoro-N-(4-{[(1-propyl-1H-imidazol-5-yl)methyl]thio}phenyl)acetamide (one-pot reaction)

Under a nitrogen atmosphere, triethylamine (27.9 ml) was added dropwise to a solution of 4-aminobenzenethiol (12.5 g) in tetrahydrofuran (180 ml) at 0 to 10°C. Subsequently, to the reaction mixture was added dropwise trifluoroacetic anhydride (28.2 ml) at 0 to 10°C and the reaction mixture was stirred at the same temperature for 1 hour. To the reaction solution was added water (30 ml) and the mixture was stirred at room temperature for 1 hour. To the solution was added dropwise triethylamine (41.8 ml) at 0 to 10°C. Subsequently, to the reaction solution was added dropwise at 0 to 20°C a solution of 5-(chloromethyl)-1-propyl-1H-imidazole hydrochloride (19.5 g) in distilled water (19 ml) and the resulting solution was stirred at the same temperature for 1 hour. Ethyl acetate (120 ml) was added to the reaction solution and the organic layer was separated. Subsequently, after washing with an aqueous 7 w/w% sodium hydrogen carbonate (60 ml) and city water (60 ml), the organic layer was concentrated. To the concentrate was added diisopropyl ether (150 ml) and the resulting mixture was stirred at 20 to 30°C for 2 hours. The precipitated crystals were collected by filtration and washed with diisopropyl ether (20 ml). The crystals were dried under reduced pressure to obtain 33.1 g (yield: 96%, calculated from the amount of 4-aminobenzenethiol) of the title compound as white crystals.

### Example 3

### 4-{[(1-Propyl-1H-imidazol-5-yl)methyl]sulfinyl}phenylamine

At 20 to 30°C, 30 w/w% hydrogen peroxide (16.4 g) was added to a solution of 2,2,2-trifluoro-N-(4-{[(1-propyl-1H-imidazol-5-yl)methyl)thio}phenyl)acetamide (33.1 g) in acetic acid (49.7 ml) and the reaction mixture was stirred at the same temperature for 3 hours. To the reaction was added ethyl acetate (330 ml), thereto were added dropwise sodium thiosulfate pentahydrate (35.9 g) and an aqueous 6 N sodium hydroxide solution (144.6 ml) at 0 to 10°C, and the reaction mixture was stirred at the same temperature for 0.5 hours. To the mixture was added tetrahydrofuran (330 ml) and the organic layer was separated. The aqueous layer was again extracted with ethyl acetate/tetrahydrofuran (160 ml/160 ml). The organic layers were combined, washed with 10w/w% brine (80 ml × 2) and concentrated. To the organic layers was added methanol (330 ml), the organic layers were dissolved therein and again concentrated.

The concentrate was dissolved in methanol (198.6 ml) and thereto was added a solution of potassium carbonate (40.0 g) in water (99.3 ml) at room temperature. The mixture was warmed to 50°C and stirred for 2.5 hours. After cooling to 20 to 30°C, the organic layer was separated and the aqueous layer was extracted with ethyl acetate (330 ml). The organic layers were combined and washed with 20 w/w% brine (100 ml), thereto were added anhydrous magnesium sulfate (5 g) and activated carbon (3 g), and the mixture was stirred at 20 to 30°C for 0.5 hours. The solids were filtered off, the filtrate was washed with ethyl acetate (64 ml) and the organic layer was concentrated. Ethyl acetate (160 ml) was added to the concentrate and the product was again concentrated. Ethyl acetate (132 ml) was added to the concentrate and the mixture was stirred at 50°C for 1 hour, and then allowed to cool to 20 to 30°C and stirred at the same temperature for 1 hour. The precipitated crystals were collected by filtration and washed with ethyl acetate (33 ml). The obtained crystals were dried under reduced pressure to obtain 18.5 g (yield: 73%) of the title compound as white crystals.
Melting point: 143°C (decomposition)
¹H-NMR (CDCl₃, 300MHz) δ: 0.90 (3H, t, J = 7.4Hz), 1.68-1.78 (2H, m), 3.74 (2H, t, J = 6.5Hz), 3.95-4.08 (4H, m), 6.60 (1H, s), 6.69 (2H, d, J = 6.8Hz), 7.17 (2H, d, J = 6.8Hz), 7.43 (1H, s)
IR (KBr, cm⁻¹) : 3397, 3334, 3216, 1650, 1596, 1419, 1018

### Example 4

### 4-{[(1-Propyl-1H-imidazol-5-yl)methyl]sulfinyl}phenylamine (one-pot reaction)

At 20 to 30°C, 30 w/w% hydrogen peroxide (35.4 g) was added to a solution of 2,2,2-trifluoro-N-(4-{[(1-propyl-1H-imidazol-5-yl)methyl]thio}phenyl)acetamide (71.5 g) in acetic acid (107.3 ml) and the reaction mixture was stirred at the same temperature for 3 hours. To the reaction mixture was added methanol (429 ml), thereto were added dropwise sodium thiosulfate pentahydrate (77.4 g) and an aqueous 6 N sodium hydroxide solution (312.2 ml) at 0 to 10°C, and the reaction mixture was stirred at the same temperature for 1 hour. Subsequently, to the mixture was added potassium carbonate (86.2 g), the resulting mixture was warmed to 50°C and stirred for 3 hours. After cooling to 20 to 30°C, the organic layer was separated and the aqueous layer was extracted with ethyl acetate (710 ml). The organic layer was washed with 20 w/w% brine (200 ml), thereto were added anhydrous magnesium sulfate (10 g) and activated carbon (7.1 g), and the mixture was stirred at 20 to 30°C for 0.5 hours. The solids were filtered off, the filtrate was washed with ethyl acetate (200 ml) and the organic layer was concentrated. Ethyl acetate (358 ml) was added to concentrate and the mixture was stirred at 50°C for 2 hours, and then allowed to cool to 20 to 30°C and stirred at the same temperature for 1 hour. The precipitated crystals were collected by filtration and washed with ethyl acetate (72 ml). The obtained crystals were dried under reduced pressure to obtain 36.3 g (yield: 66%) of the title compound as white crystals.

### Example 5

### 4-{[(1-Propyl-1H-imidazol-5-yl)methyl]sulfinyl}phenylamine

A solution of 5-(chloromethyl)-1-propyl-1H-imidazole hydrochloride (10.7 g) in distilled water (6 ml) was added dropwise to a mixed solution of 4-aminobenzenethiol (6.3 g) and triethylamine (15.3 ml) in 2-propanol (25.2 ml) at 10 to 30°C and the resulting solution was stirred under the same condition for 1 hour. To the solution was added water (20 ml) and the mixture was extracted with ethyl acetate (50 ml × 2). The organic layer was washed with 20 w/w% brine (20 ml) and concentrated.

The concentrate was dissolved in acetic acid (12.6 ml), thereto was added 30 w/w% hydrogen peroxide (8.5 g) at 20 to 30°C, and the resulting mixture was stirred at the same temperature for 2 hours. Thereto were added dropwise sodium thiosulfate pentahydrate (9.3 g) and an aqueous 6 N sodium hydroxide solution (36 ml) at 0 to 10°C, and the reaction mixture was stirred at the same temperature for 1 hour. The mixture was extracted with ethyl acetate/2-propanol (4/1, 180 ml). The organic layer was washed with 20 w/w% brine (30 ml), to the organic layer were added anhydrous sodium sulfate and activated carbon (0.6 g), and the mixture was stirred at 20 to 30°C for 1 hour. The solids were filtered off, the filtrate was washed with ethyl acetate (10 ml) and the organic layer was concentrated. To the concentrate was added 2-propanol (18 ml) and the concentrate was dissolved therein, and then the resulting solution was stirred at 20 to 30°C for 0.5 hours. Subsequently, to the solution was added n-heptane (36 ml) and the mixture was stirred at 20 to 30°C for 1 hour. The precipitated crystals were collected by filtration and washed with 2-propanol/n-heptane (4 ml/2 ml). The obtained crystals were dried under reduced pressure to obtain 10.2 g (yield: 73%) of the title compound as white crystals.

### Example 6

### 4-{[(1-Propyl-1H-imidazol-5-yl)methyl]sulfinyl}phenylamine (one-pot reaction)

A solution of 5-(chloromethyl)-1-propyl-1H-imidazole hydrochloride (0.78 g) in distilled water (0.5 ml) was added dropwise to a mixed solution of 4-aminobenzenethiol (0.46 g) and triethylamine (1.1 ml) in methanol (2 ml) at 10 to 30°C and the resulting solution was stirred under the same conditions for 1 hour. Subsequently, to the solution were added acetic acid (1 ml) and 30 w/w% hydrogen peroxide (0.62 g) at 20 to 30°C, and the resulting mixture was stirred at the same temperature for 17 hours. Thereto were added dropwise sodium sulfite (0.69 g) and an aqueous 6 N sodium hydroxide solution (3 ml) at 0 to 10°C, and the reaction mixture was stirred at the same temperature for 1 hour. The mixture was extracted with ethyl acetate/2-propanol. The organic layer was washed with 20 w/w% brine, to the organic layer were added anhydrous sodium sulfate and activated carbon (40 mg), and the product was stirred at 20 to 30°C for 1 hour. The solids were filtered off, the filtrate was washed with ethyl acetate (10 ml) and the organic layer was concentrated. To the concentrate was added ethyl acetate (4 ml) and the concentrate was dissolved therein, and then the resulting solution was stirred at 20 to 30°C for 1 hour. The precipitated crystals were collected by filtration and washed with ethyl acetate (2 ml). The obtained crystals were dried under reduced pressure to obtain 0.73 g (yield: 71%) of the title compound as white crystals.

### Example 7

### (-)-4-[[(1-Propyl-1H-imidazol-5-yl)methyl]sulfinyl]phenylamine(2S, 3S)-di(1-naphthoyl)tartrate

The racemate (50 mg) of 4-[[(1-propyl-1-H-imidazol-5-yl)methyl]sulfinyl]phenylamine and (2S, 3S)-di(1-naphthoyl)tartaric acid monohydrate (45.2 mg) were dissolved in methanol (1.0 ml) and the resulting solution was stirred at room temperature overnight. The precipitate was filtered to obtain 56.2 mg of the crystals. As a result of HPLC analysis, the excess rate of the diastereomers was 88%de. These crystals (55 mg) were heated under reflux in ethanol (1.5 ml) for 0.5 hours and the mixture was stirred at room temperature overnight. The precipitate was filtered to obtain 48.1 mg of the crystals. As a result of HPLC analysis, the excess rate of the diastereomers was 95%de. These crystals (47 mg) were heated under reflux in methanol (2 ml) and water (1 ml) for 0.5 hours and the mixture was stirred at room temperature overnight. The precipitate was filtered to obtain 41.8 mg of the crystals. As a result of HPLC analysis, the excess rate of the diastereomers was 99%de.
Specific rotation; [α]²⁷_{D} = -45.5 (c = 0.2 MeOH)
Melting point: 178°C (decomposition)

| Elemental analysis value: in terms of C₁₃H₁₇N₃OS · C₂₆H₁₈O₈ | | | | |
|---|---|---|---|---|
| Calcd. value | C, 64.90; | H, 4.89; | N, 5.84; | S, 4.44 |
| Found value | C, 64.65; | H, 4.63; | N, 5.65; | S, 4.31 |

### Example 8

### (-)-4-{[(1-Propyl-1H-imidazol-5-yl)methyl]sulfinyl}phenylamine

The racemate (200 mg) of (-)-4-{[(1-propyl-1H-imidazol-5-yl)methyl]sulfinyl}phenylamine and (2S, 3S)-di(1-naphthoyl)tartaric acid monohydrate (180.8 mg) were heated under reflux in ethyl acetate (1.5 ml) and methanol (4 ml) for about 0.5 hours and the resulting solution as such was allowed to stand at room temperature. The precipitate was filtered to obtain 223.6 mg of the crystals. As a result of HPLC analysis, the excess rate of the diastereomers was 92%de. These crystals (223 mg) were heated under reflux in methanol (13 ml) for 0.5 hours and the mixture was stirred at room temperature overnight. The precipitate was filtered to obtain 188.1 mg of the crystals. As a result of HPLC analysis, the excess rate of the diastereomers was 99%de. These crystals (187 mg) were metathesized in saturated sodium bicarbonate solution (5 ml) and water (5 ml) and the mixture was extracted three times with chloroform (about 15 ml). The chloroform layer was dried over anhydrous magnesium sulfate, and then concentrated to obtain 62.4 mg (yield: 31%) of the crystals. As a result of HPLC analysis, the excess rate of the enantiomers was 99%ee.
H¹-NMR (DMSO-d₆) δ; 0.78-0.82 (3H, t, J = 7.3Hz), 1.58-1.67 (2H, m), 3.72-3.76 (2H, t, J = 7.0Hz), 4.05-4.14 (2H, m), 5.71 (2H, s), 6.54 (1H, s), 6.61-6.63 (2H, d, J = 7.6Hz), 7.14-7.16 (2H, d, J = 7.6Hz), 7.59 (1H, s)
Melting point: 137 - 138°C

### Example 9

### (-)-4-{[(1-Propyl-1H-imidazol-5-yl)methyl]sulfinyl}phenylamine · di-p-toluoyl-D-tartrate · monohydrate

To a mixed solution of (2S,3S)-2,3-bis[(4-methylbenzoyl)oxy]butane dicarboxylic acid (15.1 g) and 4-{[(1-propyl-1H-imidazole-5-yl)methyl]sulfinyl}phenylamine (10.3 g) in 1,2-dimethoxyethane (90 ml) was added dropwise water (90 ml) and the resulting mixture was stirred at room temperature overnight. The precipitated crystals were collected by filtration, washed with 50 v/v% aqueous 1,2-dimethoxyethane (30 ml), and dried under reduced pressure. The crystals were dissolved in 50v/v% aqueous acetonitrile (84 ml) with heating at 70°C and thereto was added water (42 ml) while keeping at the same temperature. The mixture was allowed to cool to room temperature, and stirred at room temperature overnight and subsequently at 0°C for 1 hour. The precipitated crystals were collected by filtration and washed with 75% aqueous acetonitrile (30 ml) that was cooled to 0°C. The obtained crystals were dried under reduced pressure to obtain 10.9 g (yield: 41.6%, 99.6%de) of the title compound as white crystals.
Melting point: 134 - 136°C

| Elemental analysis value: in terms of C₃₃H₃₅N₃O₉S ·1 H₂O | | | | |
|---|---|---|---|---|
| Theoretical | value: C, 59.36; | H, 5.59; | N, 6.29; | S, 4.80 |
| Analytical | value: C, 59.26; | H, 5.67; | N, 6.18; | S, 4.77 |

### Example 10

### (-)-4-{[(1-Propyl-1H-imidazol-5-yl)methyl]sulfinyl}phenylamine

(-)-4-{[(1-Propyl-1H-imidazol-5-yl)methyl]sulfinyl}phenylamine · di-p-toluoyl-D-tartrate · monohydrate (5 g) was dissolved in a mixture of 3 N hydrochloric acid (10 ml) and ethyl acetate (20ml) and the aqueous layer was separated. The aqueous layer was adjusted to pH 9 with 6 N aqueous sodium hydroxide solution (5 ml), crystallized by adding seed crystals and the mixture was stirred at room temperature. The crystals were collected by filtration to obtain 1.88 g (yield: 95.4%) of the title compound as white powder.

### Example 11

### (-)-4-{[(1-Propyl-1-H-imidazol-5-yl)methyl]sulfinyl}phenylamine and (R)-N-(3,5-dinitrobenzoyl)phenylglycine salt

The racemate (50 mg) of 4-[[(1-propyl-1-H-imidazol-5-yl)methyl]sulfinyl]phenylamine and (R)-N-(3,5-dinitrobenzoyl)phenylglycine (65.6 mg) were dissolved in methanol (1.0 ml) and the resulting solution was allowed to stand at room temperature overnight. The precipitate was filtered to obtain 57.9 mg of the crystals. As a result of HPLC analysis, the excess rate of the diastereomers was 51%de. These crystals (57 mg) were dissolved in ethanol (1.5 ml) and the product was stirred at room temperature overnight. The precipitate was filtered to obtain 27.9 mg of the crystals. As a result of HPLC analysis, the excess rate of the diastereomers was 80%de.

### Example 12

### (-)-4-{[(1-Propyl-1H-imidazol-5-yl)methyl]sulfenyl}phenylamine · di-p-toluoyl-D-tartrate · monohydrate

4-Aminothiophenol (2.5 g) was dissolved in water (2.5 ml) and isopropanol (10 ml), to the resulting solution was added triethylamine (5.5 ml) and the mixture was cooled to - 15 to -10°C.

To the mixture was added dropwise a solution of 5-(chloromethyl)-1-propyl-1H-imidazole hydrochloride (3.9 g) in water (2.5 ml) at -15 to -10°C and the resulting mixture was stirred at the same temperature for 1 hour. The isopropanol was distilled off under reduced pressure and to the residue was added methyl isobutyl ketone (25 ml) and the organic layer was washed with water. Activated carbon (0.1 g) was added to the organic layer and the mixture was stirred at room temperature for 10 minutes. The organic layer was concentrated and the concentrate was dissolved in methyl isobutyl ketone (30 ml).

Separately, di-p-toluoyl-(D)-tartaric acid (7.7 g) was dissolved in a mixed solution of toluene (90 ml) and methyl isobutyl ketone (60 ml) and to the resulting solution was added water (3.6 ml). Subsequently, to the solution was slowly added dropwise the above-mentioned methyl isobutyl ketone solution over 2 hours. After stirring for 1 hour, to the mixture was added 30% hydrogen peroxide (6.8 g) and the resulting mixture was stirred at room temperature for 24 hours. To the mixture was added methanol (30 ml) and the resulting solution was stirred at 50°C for 8 hours. To the solution was added water (30 ml) and the resulting mixture was stirred at room temperature for 5 hours. The precipitated crystals were collected by filtration and washed with water (30 ml) to obtain 7.1 g (yield: 53%) of the title compound.

### Example 13

### (-)-7-[4-(2-Butoxyethoxy)phenyl]-1-isobutyl-N-(4-{[(1-propyl-1H-imidazol-5-yl)methyl]sulfinyl}phenyl)-2,3-dihydro-1H-1-benzazepine-4-carboxamide

(-)-4-{[(1-Propyl-1H-imidazol-5-yl)methyl]sulfinyl}phenylamine · di-p-toluoyl-D-tartrate · monohydrate (5 g) was dissolved in a mixture of 1 N hydrochloric acid (25 ml) and ethyl acetate (15 ml) and the aqueous layer was separated. The aqueous layer were made pH 9 by adding an aqueous 25% potassium carbonate (25 ml) and extracted three times with ethyl acetate - 2-propanol (4 : 1) (25 ml). The organic layer was washed with saturated brine (25 ml) and dried over magnesium sulfate. The solvent was distilled off to obtain (-)-4-{[(1-propyl-1H-imidazol-5-yl)methyl]sulfinyl}phenylamine.

Separately, to a solution of 7-[4-(2-butoxyethoxy)phenyl]-2-isobutyl-2,3-dihydro-2H-1-benzazepine-4-carboxylic acid (2.56 g) in tetrahydrofuran (7.5 ml) was added N,N-dimethylformamide (one drop), and added dropwise oxalyl chloride (0.56 ml) at room temperature and the resulting solution was stirred for 1 hour to prepare an acid chloride.

To a solution of (-)-4-{[(1-propyl-1H-imidazol-5-yl)methyl]sulfinyl}phenylamine in tetrahydrofuran (17.5 ml) was added triethylamine (2.85 ml)(the solution was almost clear) and added dropwise an acid chloride solution at room temperature, and the resulting solution was stirred for 1 hour. To the solution was added water (15 ml) and the mixture was extracted with ethyl acetate. The extract was sequentially washed with an aqueous 10% acetic acid solution, saturated sodium bicarbonate solution and 10% brine, and thereto were added basic silica gel (4 g), activated carbon (0.4 g) and sodium sulfate (2 g) and the mixture was stirred for 10 minutes and filtered, and the solvent was distilled off. To the residue was added tert-butyl methyl ether (20 ml) and added water (4 ml), and the resulting mixture was stirred at room temperature for 1 hour. The precipitated crystals were collected by filtration to obtain tert-butyl methyl ether solvate (3.62 g, 80.3%) of the title compound. To the resulting product was added ethanol (3.5 ml) and dissolved therein at 40°C, and thereto was added tert-butyl methyl ether (31.5 ml) and the mixture was stirred for 14 hours under room temperature and stirred for 1 hour under ice-cooling. The crystals were collected by filtration to obtain tert-butyl methyl ether solvate (3.52 g, 78%, 99%ee) of the title compound as yellow powder.
¹H-NMR (CDCl₃, 300MHz) δ; title compound : tert-butyl methyl ether = 1 : 0.94; title compound: 0.84-0.97 (12H, m), 1.28-1.42 (2H, m), 1.53-1.75 (4H, m) 2.02-2.11 (1H, m), 2.88-2.94 (2H, m), 3.17-3.21 (2H, m), 3.33-3.37 (2H, m), 3.53 (2H, t, J = 6.6Hz), 3.71-3.81 (4H, m), 3.95-4.10 (2H, m), 4.13-4.16 (2H, m), 6.55 (1H, s), 6.90-6.98 (3H, m), 7.32 (2H, d, J = 8.7Hz), 7.43-7.47 (5H, m), 7.75 (2H, d, J = 8.7Hz), 8.32 (1H, s), NH undetected. tert-butyl methyl ether: 1.19 (9H, s), 3.21 (3H, s).

### Example 14

### (-)-7-[4-(2-Butoxyethoxy)phenyl]-1-isobutyl-N-(4-{[(1-propyl-1H-imidazol-5-yl)methyl]sulfinyl}phenyl)-2,3-dihydro-1H-1-benzazepine-4-carboxamide

To a solution of 7-[4-(2-butoxyethoxy)phenyl]-1-isobutyl-2,3-dihydro-1H-1-benzazepine-4-carboxylic acid (2.56 g) in tetrahydrofuran (8 ml) was added N,N-dimethylformamide (one drop), and added dropwise oxalyl chloride (0.56 ml) under ice-cooling and the resulting solution was stirred for 1 hour to prepare an acid chloride.

To a solution of (-)-4-{[(1-propyl-1H-imidazol-5-yl)methyl]sulfinyl}phenylamine in tetrahydrofuran (18 ml) was added diisopropylethylamine (3.5 ml) and added dropwise the acid chloride solution at 10°C or lower, and the resulting solution was stirred for 2 hours. To the solution was added water (15 ml) and the mixture was extracted with ethyl acetate. The organic layer was sequentially washed with 10% aqueous acetic acid solution, saturated sodium bicarbonate solution and 10% brine, and thereto were added basic silica gel (4 g), activated carbon (0.4 g) and sodium sulfate (2 g) and the mixture was stirred for 10 minutes and filtered, and the solvent was distilled off. To the residue was added isopropyl acetate (15 ml) and the residue was dissolved therein with stirring at 40°C, and the resulting solution was stirred for 14 hours at room temperature. To the solution was added heptane (15 ml) and the mixture was stirred for 1 hour at room temperature, and then ice-cooled. The crystals were collected by filtration to obtain isopropyl acetate solvate (2.93 g) of the title compound. Subsequently, thereto was added isopropyl acetate (10 ml). After dissolution at 40°C, the solution was stirred for 4 hours at room temperature and stirred for 1 hour under ice-cooling. The crystals were collected by filtration, washed with isopropyl acetate (15 ml) and dried under reduced pressure to obtain isopropyl acetate solvate (2.8 g, 77.1%) of the title compound as yellow powder.
¹H-NMR (CDCl₃, 300MHz) δ; title compound : isopropyl acetate = 1 : 0.80; title compound: 0.84-0.97 (12H, m), 1.28-1.42 (2H, m), 1.53-1.75 (4H, m) 2.02-2.11 (1H, m), 2.88-2.94 (2H, m), 3.17-3.21 (2H, m), 3.33-3.37 (2H, m), 3.53 (2H, t, J = 6.6Hz), 3.71-3.81 (4H, m), 3.95-4.10 (2H, m), 4.13-4.16 (2H, m), 6.55 (1H, s), 6.90-6.98 (3H, m), 7.32 (2H, d, J = 8.7Hz), 7.43-7.47 (5H, m), 7.75 (2H, d, J = 8.7Hz), 8.32 (1H, s), isopropyl acetate: 1.27 (6H, d, J = 6.3Hz), 2.06 (3H, s), 4.99-5.08 (1H, m).

### Example 15

### (-)-8-[4-(2-Butoxyethoxy)phenyl]-1-isobutyl-N-(4-{[(1-propyl-1H-imidazol-5-yl)methyl]sulfinyl}phenyl)-1,2,3,4-tetrahydro-1-benzazocine-5-carboxamide · methanesulfonate

8-[4-{2-Butoxyethoxy)phenyl]-1-isobutyl-1,2,3,4-tetrahydro-1-benzazocine-5-carboxylic acid (986 mg) was dissolved in tetrahydrofuran (3 ml) and thereto was added N,N-dimethylformamide (one drop). Subsequently, to the resulting solution was added dropwise oxalyl chloride (0.2 ml, 2.29 mmol) under ice-cooling and the mixture was stirred for 80 minutes under ice-cooling to prepare an acid chloride.

Separately, (-)-4-{[(1-propyl-2H-imidazol-5-yl)methyl]sulfinyl}phenylamine (689 mg) was added to tetrahydrofuran (7 ml) and the resulting solution was cooled to 5°C. To the solution was added dropwise pyridine (0.62 ml) and added dropwise the acid chloride solution at 3 to 5°C, and the mixture was stirred for 2 hours under ice-cooling. To the mixture was added water (20 ml) at 10°C or lower and the mixture was extracted with ethyl acetate. The organic layer was sequentially washed with water, saturated sodium bicarbonate solution and water, and concentrated under reduced pressure. Thereto was added toluene and the mixture was concentrated under reduced pressure. Thereto was added acetonitrile and the mixture was concentrated under reduced pressure. The residue was dissolved in acetonitrile (7 ml) and acetone (7 ml), thereto was added dropwise methanesulfonic acid (209 mg), and added seed crystals and the mixture was stirred at room temperature for 100 minutes. Subsequently, to the mixture was added acetone - acetonitrile (1 : 1, 5 ml). After stirring at room temperature overnight, the mixture was stirred for 2.5 hours under ice-cooling. The precipitated crystals were collected by filtration and washed with the ice-cooled acetone (9 ml). The crystals were dried at 40°C under reduced pressure to obtain 1.51 g (yield: 87%) of the title compound as yellow crystals.
¹H-NMR (300MHz, DMSO-d₆, δ): 0.78-0.96 (12H, m), 1.25-1.40 (2H, m), 1.41-1.51 (4H, m), 1.65-1.85 (2H, m), 2.05-2.15 (1H, m), 2.30 (3H, s), 2.35-2.50 (2H, m), 3.05-3.15 (2H, m), 3.30-3.55 (4H, m), 3.65-3.70 (2H, m), 3.90-4.05 (2H, m), 4.05-4.10 (2H, m), 4.30 (1H, d, J = 14.73Hz), 4.65 (1H, d, J = 14.73Hz), 6.85 (1H, d, J = 8.97Hz), 6.97 (1H, d, J = 8.79Hz), 7.17 (1H, s), 7.35-7.75 (6H, m), 7.92 (2H, d, J = 8.79Hz), 9.08 (1H, s), 10.15 (1H, s).

| Elemental analysis value: in terms of C₄₁H₅₂N₄O₄S · CH₄SO₃ | | | | |
|---|---|---|---|---|
| Calcd. value | C, 63.61; | H, 7.12; | N, 7.06; | S, 8.09 |
| Found value | C, 63.65; | H, 7.23; | N, 7.05; | S, 8.08 |

### Example 16

### (-)-8-[4-(2-Butoxyethoxy)]phenyl]-1-isobutyl-N-(4-{[(1-propyl-1H-imidazol-5-yl)methyl]sulfinyl)phenyl)-1,2,3,4-tetrahydro-1-benzazocine-5-carboxamide · methanesulfonate

To a solution of 8-[4-(2-butoxyethoxy)phenyl]-1-isobutyl-1,2,3,4-tetrahydro-1-benzazocine-5-carboxylic acid (5 g) in tetrahydrofuran (15 ml) was added N,N-dimethylformamide (one drop), added dropwise oxalyl chloride (1.1 ml) under ice-cooling and the mixture was stirred for 1 hour to prepare an acid chloride.

Separately, to a solution of (-)-4-{[(1-propyl-1H-imidazol-5-yl)methyl]sulfinyl}phenylamine (3.22 g) in tetrahydrofuran (35 ml) was added diisopropylethylamine (6.7 ml) and added dropwise the acid chloride solution at 10°C or lower, and the resulting mixture was stirred for 1 hour. To the mixture were added water (50 ml) and toluene (50 ml), and the mixture was adjusted to about pH 4 by adding acetic acid (about 8 ml) and separated into layers. The organic layer was washed with saturated sodium bicarbonate solution to adjust to pH 7 to 8, washed with 10% brine, and then thereto were added basic silica gel (4 g), activated carbon (0.5 g) and sodium sulfate (2 g) and the mixture was stirred for 10 minutes and filtered, and washed with toluene (20 ml). The solvent was distilled off and the residue was dissolved in methyl isobutyl ketone (15 ml) and thereto was added methanesulfonic acid (0.65 ml), and added seed crystals (80 mg) and the mixture was stirred for 16 hours. To the mixture was added methyl isobutyl ketone - ethyl acetate (1 : 1, 50 ml), and the resulting mixture was stirred for 2 hours under ice-cooling. The crystals were collected by filtration and dried at 40°C under reduced pressure to obtain 6.62 g of yellow powder. These crystals were suspended in methyl isobutyl ketone (40 ml) and the suspension was stirred for 16 hours, and then thereto was added ethyl acetate (40 ml) and the resulting solution was stirred at room temperature for 1 hour and stirred for 2 hours under ice-cooling. The crystals were collected by filtration to obtain 6.05 g (yield: 68.7%) of the title compound as yellow powder.

### Example 17

### (-)-4-{[(1-Propyl-1H-imidazol-5-yl)methyl]sulfinyl}phenylamine · di-p-toluoyl-D-tartrate · monohydrate

To a solution of di-p-toluoyl-D-tartaric acid (1.9 g) in toluene (15 ml) and methyl isobutyl ketone (30 ml) was added a solution of 4-{[(1-propyl-1H-imidazol-5-yl)methyl]thio}phenylamine (1.2 g) in methyl isobutyl ketone (15 ml). Subsequently, to the resulting mixture was added 30% hydrogen peroxide (1.7 g) and the mixture was stirred at room temperature for 3 weeks. The precipitated crystals were collected by filtration. The obtained crystals were dried until they became constant weight to obtain 2.9 g (yield: 87%, 82.7%de) of the title compound. The crystals were added to acetonitrile/water (9 ml/9 ml) and the mixture was stirred at 60°C for 0.5 hours, and thereto was added dropwise water (9 ml). The mixture was stirred at the same temperature for 0.5 hours, stirred at room temperature for 1 hour, and stirred for 1 hour under ice-cooling. The precipitated crystals were collected by filtration and washed with ice-cooled acetonitrile/water (4 ml/2 ml). The obtained crystals were dried until they became constant weight to obtain 2.4 g (yield: 72%, 98.1%de) of the title compound.

### Example 18

### (+)-4-([(1-Propyl-1H-imidazol-5-yl)methyl]sulfinyl)phenylamine

To a mixture of 4-{[(1-propyl-1H-imidazol-5-yl)methyl]thio}phenylamine (0.99 g) and (R)-(-)-1,1'-binapthyl-2,2'-diyl hydrogenphosphate (0.14 g) in dichloromethane (5 ml) was added 30% hydrogen peroxide (0.14 g) and the mixture was stirred at room temperature for 6 hours. A portion of the reaction solution was sampled and analyzed by high performance liquid chromatography (HPLC). Conversion rate: 52%, Optical purity: 35.0%ee.

### HPLC condition:

Column: Chiralcel (Daicel) OD
Mobile phase: Hexane - ethanol (85 : 15)
Flow rate: 1 ml/min
Temperature: 35°C
(+)-isomer: 21 min, (-)-isomer: 27 min

### Example 19

### (-)-8-[4-(2-Butoxyethoxy)phenyl]-1-isobutyl-N-(4-{[(1-propyl-1H-imidazol-5-yl)methyl]sulfinyl}phenyl)-1,2,3,4-tetrahydro-1-benzazocine-5- carboxamide · methanesulfonate

To a solution of 8-[4-(2-butoxyethoxy)phenyl]-1-isobutyl-1,2,3,4-tetrahydro-1-benzazocine-5-carboxylic acid (90 g) in tetrahydrofuran (7.5 ml) was added N,N-dimethylformamide (460 mg) and then added dropwise thionyl chloride (24.9 g) at 10 to 15°C, and the resulting solution was stirred at the same temperature for 40 minutes to prepare an acid chloride.

Separately, to a solution of (-)-4-{[(1-propyl-1H-imidazol-5-yl)methyl]sulfinyl}phenylamine in tetrahydrofuran (540 ml) was added pyridine (55.18 g), the resulting solution was adjusted to 5°C or lower, and then thereto was added dropwise the acid chloride solution at 5°C or lower, and the resulting mixture was stirred at the same temperature for 2.8 hours. To the mixture were added water (540 ml) and 20% aqueous citric acid solution (360 ml), tetrahydrofuran was distilled off under reduced pressure and the residue was extracted with ethyl acetate. The extract was sequentially washed with water, saturated sodium bicarbonate solution and water, and then the solvent was distilled off. To the residue was added acetonitrile (720 ml) and ethyl acetate (720 ml) and added dropwise methanesulfonic acid (18.2 g), and the resulting mixture was stirred at room temperature for 1 hour. The precipitated crystals were collected by filtration to obtain 141.8 g (yield: 94.4%) of the title compound as yellow crystals.

### Example 20

### (-)-8-[4-(2-Butoxyethoxy)phenyl]-1-propyl-N-(4-{[(1-propyl-1H-imidazol-5-yl)methyl]sulfinyl}phenyl)-1,2,3,4-tetrahydro-1-benzazocine-5-carboxamide · methanesulfonate

According to the same method as that described in Example 15, the title compound was produced from 8-[4-(2-butoxyethoxy)phenyl]-1-propyl-1,2,3,4-tetrahydro-1-benzazocine-5-carboxylic acid and (-)-4-{[(1-propyl-1H-imidazol-5-yl)methyl]sulfinyl}phenylamine.
¹H-NMR (CDCl₃, δ, 300MHz) 0.88-1.01 (9H, m), 1.37-1.42 (2H, m), 1.57-1.80 (8H, m), 2.63 (2H, br), 2.77 (3H, s), 3.27 (2H, br), 3.51-3.57 (4H, m), 3.77-3.86 (4H, m), 3.90-4.05 (1H, m), 4.14 (2H, t, J = 4.6Hz), 4.25 (1H, d, J = 14.6Hz), 6.73 (1H, s), 6.84 (1H, d, J = 8.7Hz), 6.93 (2H, d, J = 8.8Hz), 7.21 (2H, d, J = 8.7Hz), 7.40-7.48 (4H, m), 7.61 (1H, s), 7.89 (2H, d, J = 8.7Hz), 8.65 (1H, s), 9.27 (1H, br)

| Elemental analysis value: in terms of C₄₁H₅₄N₄O₇S₂ | | | | |
|---|---|---|---|---|
| Calcd. value | C, 63.21; | H, 6.99; | N, 7.19; | S, 8.23 |
| Analytical value | C, 63.00; | H, 7.09; | N, 7.41; | S, 8.25 |

### Example 21

### (-)-8-[4-(2-Butoxyethoxy)phenyl]-1-isobutyl-N-(4-{[(1-propyl -1H-imidazol-5-yl)methyl]sulfinyl}phenyl)-1,2,3,4-tetrahydro-1-benzazocine-5-carboxamide

To a solution of 8-[4-(2-butoxyethoxy)phenyl]-1-isobutyl-1,2,3,4-tetrahydro-1-benzazocine-5-carboxylic acid (45 g) in tetrahydrofuran (135 ml) was added N,N-dimethylformamide (230 mg) and added dropwise thionyl chloride (12.45 g) at 10 to 15°C, and the resulting solution was stirred at the same temperature for 40 minutes to prepare an acid chloride.

Separately, to a solution of (-)-4-{[(1-propyl-1H-imidazol-5-yl)methyl]sulfinyl}phenylamine in tetrahydrofuran (270 ml) was added pyridine (27.59 g), the resulting mixture was adjusted to 5°C or lower, and then thereto was added dropwise the acid chloride solution at 5°C or less, and the resulting mixture was stirred at the same temperature for 2 hours. To the mixture were added water (270 ml) and 20% aqueous citric acid solution (180 ml), tetrahydrofuran was distilled off under reduced pressure and the residue was extracted with ethyl acetate. The extract was sequentially washed with water, saturated sodium bicarbonate solution and water, and then the solvent was distilled off. To the residue was added ethyl acetate (360 ml), added heptane (360 ml) at 40°C and added seed crystals of (-)-8-[4-(2-butoxyethoxy)phenyl]-1-isobutyl-N-(4-{[(1-propyl-1H-imidazol-5-yl)methyl]sulfinyl}phenyl)-1,2,3,4-tetrahydro-1-benzazocine-5-carboxamide (10 mg), and the mixture was stirred at 25°C for 2 hours and stirred at 5°C for 1 hour. The precipitated crystals were collected by filtration to obtain 63.97 g (yield: 92.1%) of the title compound. Melting point: 120 - 122°C.

| Elemental analysis value: in terms of C₄₁H₅₂N₄O₄S | | | |
|---|---|---|---|
| Calcd. value | C, 70.66; | H, 7.52; | N, 8.04 |
| Analytical value | C, 70.42; | H, 7.52; | N, 8.01 |

### Industrial Applicability

According to the present invention, an optically active sulfoxide derivative having CCR5 antagonism or an intermediate compound thereof can be prepared without causing side reactions such as racemization and Pummerer rearrangement. In particular, Process 7 is industrially advantageous since it is possible to prepare an optically active Compound (II) by asymmetric oxidization in the presence of an optically active acid.

## Claims

1. A process for preparing an optically active compound represented by the formula (I): 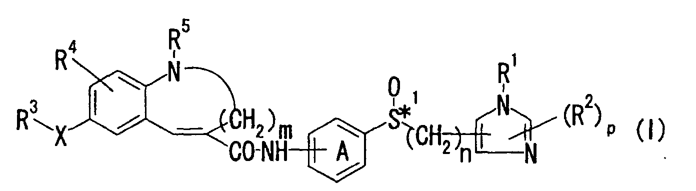 wherein R¹ represents a hydrogen atom, an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
R³ represents an optionally substituted 5- or 6-membered ring; R⁴ represents a hydrogen atom, an optionally substituted lower alkyl group, an optionally substituted lower alkoxy group or a halogen atom;
R⁵ represents a hydrogen atom, an optionally substituted hydrocarbon group, an optionally substituted heterocyclic group, an optionally substituted sulfonyl group, an esterified or amidated carboxyl group or an optionally substituted acyl group;
X represents a bond or a divalent group containing a linear part constituted of 1 to 4 atoms;
the ring A represents a benzene ring which may be substituted with a halogen atom, a C₁₋₄ alkyl group which may be substituted with a halogen atom or a C₁₋₄ alkoxy group which may be substituted with a halogen atom;
m is an integer of 1 to 5;
n represents an integer of 0 to 3;
p represents an integer of 0 to 2; and
^{*1} represents an asymmetric center,
or a salt thereof, which comprises reacting an optically active compound represented by the formula (II): 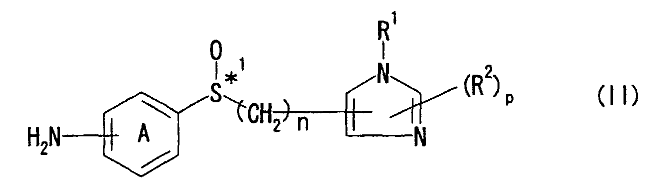 wherein each symbol is as defined above,
or a salt thereof, with a compound represented by the formula (III): 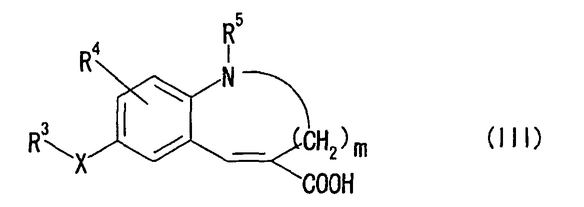 wherein each symbol is as defined above, a salt thereof, or a reactive derivative thereof.

2. A process for preparing an optically active compound represented by the formula (I): 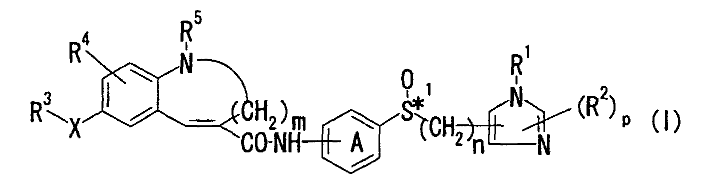 wherein R¹ represents a hydrogen atom, an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
R³ represents an optionally substituted 5- or 6-membered ring;
R⁴ represents a hydrogen atom, an optionally substituted lower alkyl group, an optionally substituted lower alkoxy group or a halogen atom;
R⁵ represents a hydrogen atom, an optionally substituted hydrocarbon group, an optionally substituted heterocyclic group, an optionally substituted sulfonyl group, an esterified or amidated carboxyl group or an optionally substituted acyl group;
X represents a bond or a divalent group containing a linear part constituted of 1 to 4 atoms;
the ring A represents a benzene ring which may be substituted with a halogen atom, a C₁₋₄ alkyl group which may be substituted with a halogen atom or a C₁₋₄ alkoxy group which may be substituted with a halogen atom;
m is an integer of 1 to 5;
n represents an integer of 0 to 3;
p represents an integer of 0 to 2; and
^{*1} represents an asymmetric center,
or a salt thereof, which comprises reacting an optically active compound represented by the formula (XIa): 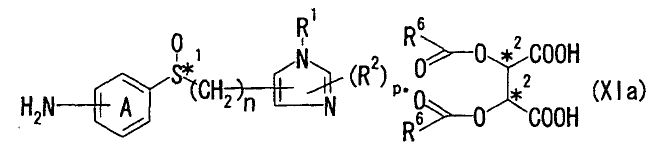 wherein R⁶ represents a methyl group, a phenyl group, a 4-methylphenyl group or a α-naphthyl group;
^{*2} represents an asymmetric center; and
the other symbols are as defined above,
or an optically active compound represented by the formula (XIb): 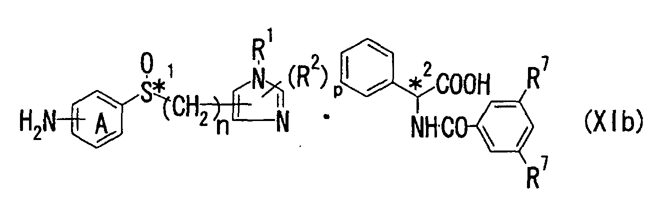 wherein R⁷ represents a hydrogen atom, a chlorine atom or a nitro group; and
the other symbols are as defined above,
with a compound represented by the formula (III): 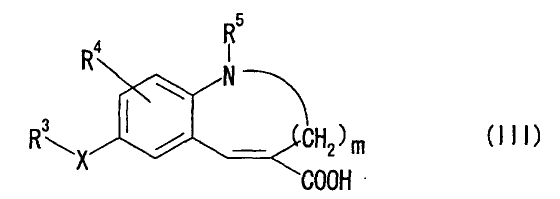 wherein each symbol is as defined above, a salt thereof or a reactive derivative thereof.

3. An optically active compound represented by the formula (II): 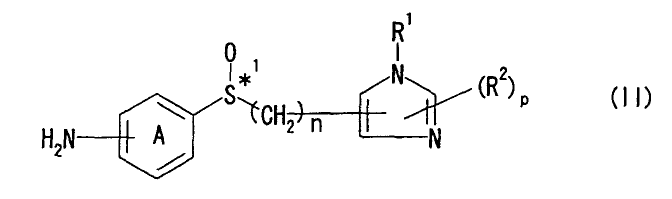 wherein R¹ represents a hydrogen atom, an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3;
p represents an integer of 0 to 2; and
^{*1} represents an asymmetric center, or a salt thereof.

4. The optically active compound according to claim 3, wherein R¹ and R² each represents a C₁₋₆ alkyl group; and n represents 1 or 2, or a salt thereof.

5. The optically active compound according to claim 3, wherein R¹ represents a C₁₋₆ alkyl group; p represents 0; n represents 1; and 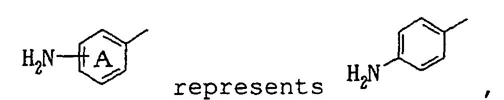 or a salt thereof.

6. A process for preparing an optically active compound represented by the formula (II): 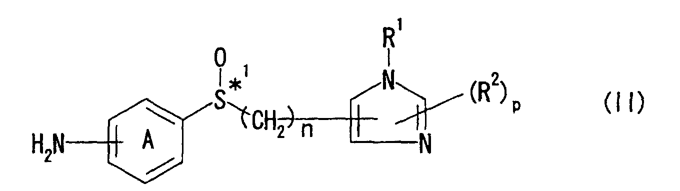 wherein R¹ represents a hydrogen atom, an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3;
p represents an integer of 0 to 2; and
^{*1} represents an asymmetric center,
or a salt thereof, which comprises subjecting an optically active compound represented by the formula (XIa): 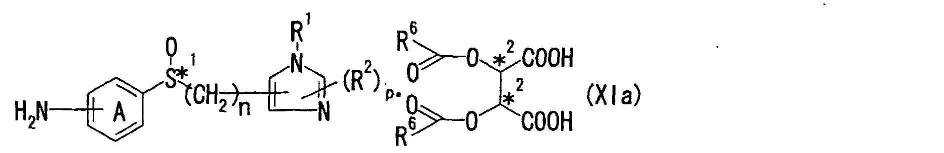 wherein R⁶ represents a methyl group, a phenyl group, a 4-methylphenyl group or a α-naphthyl group;
^{*2} represents an asymmetric center; and
the other symbols are as defined above,
or an optically active compound represented by the formula (XIb) : 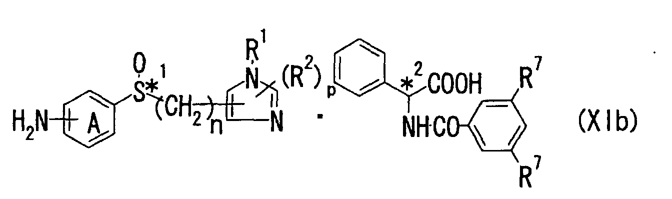 wherein R⁷ represents a hydrogen atom, a chlorine atom or a nitro group; and
the other symbols are as defined above,
to a metathesis reaction.

7. An optically active compound represented by the formula (XIa): 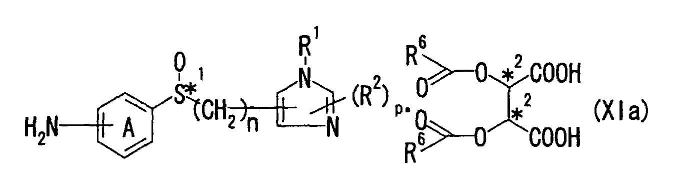 wherein R¹ represents a hydrogen atom, an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
R⁶ represents a methyl group, a phenyl group, a 4-methylphenyl group or a α-naphthyl group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3;
p represents an integer of 0 to 2; and
^{*1} and ^{*2} each represents an asymmetric center,
or an optically active compound represented by the formula (XIb): 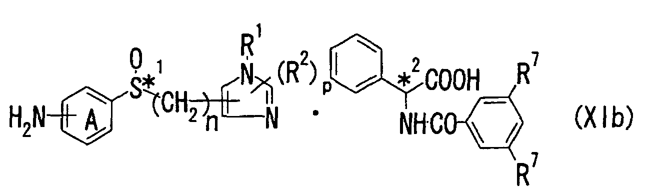 wherein R⁷ represents a hydrogen atom, a chlorine atom or a nitro group; and
the other symbols are as defined above.

8. The optically active compound according to 7, wherein R¹ and R² each represents a C₁₋₆ alkyl group; n represents 1 or 2; and R⁶ represents a 4-methylphenyl group or R⁷ represents a nitro group.

9. The optically active compound according to 7, wherein R¹ represents a C₁₋₆ alkyl group; p represents 0; n represents 1; 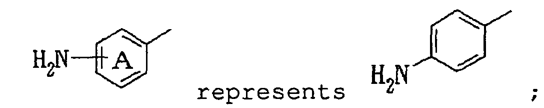 and R⁶ represents a 4-methylphenyl group or R⁷ represents a nitro group.

10. A process for preparing an optically active compound represented by the formula (XIa): 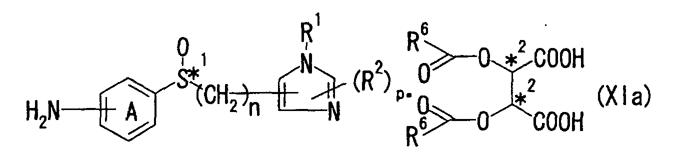 wherein R¹ represents a hydrogen atom, an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
R⁶ represents a methyl group, a phenyl group, a 4-methylphenyl group or a α-naphthyl group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3;
p represents an integer of 0 to 2; and
*¹ and ^{*2} each represents an asymmetric center,
or the formula (XIb): 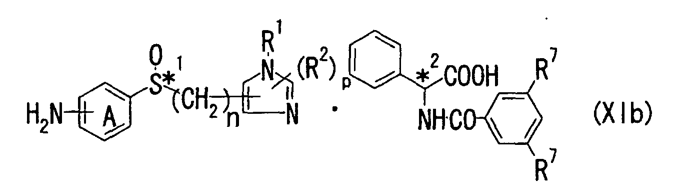 wherein R⁷ represents a hydrogen atom, a chlorine atom or a nitro group; and
the other symbols are as defined above,
which comprises subjecting a compound represented by the formula (IX): 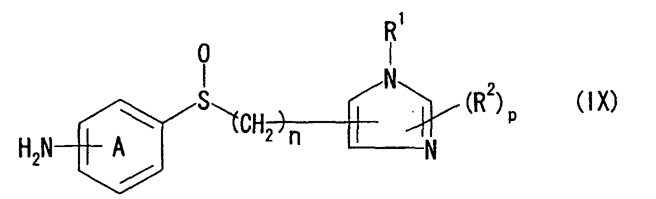 wherein each symbol is as defined above,
or a salt thereof, to optical resolution with an optically active compound represented by the formula (XIIa): 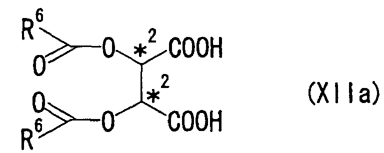 wherein each symbol is as defined above,
or an optically active acid represented by the formula (XIIb): 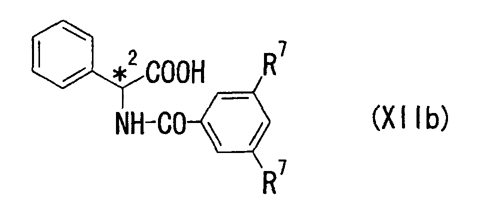 wherein each symbol is as defined above.

11. A process for preparing an optically active compound represented by the formula (II): 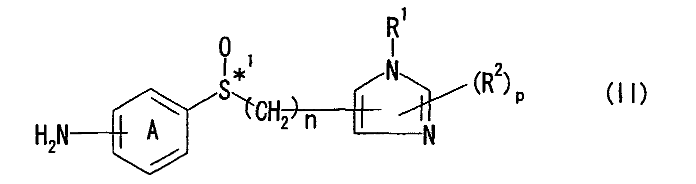 wherein R¹ represents a hydrogen atom, an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3;
p represents an integer of 0 to 2; and
^{*1} represents an asymmetric center,
or a salt thereof, which comprises oxidizing a compound represented by the formula (X): 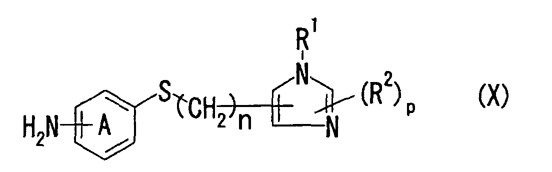 wherein each symbol is as defined above,
or a salt thereof in the presence of an optically active compound represented by the formula (XIIa): 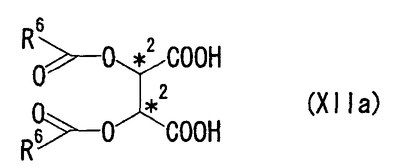 wherein R⁶ represents a methyl group, a phenyl group, a 4-methylphenyl group or a α-naphthyl group; and
^{*2} represents an asymmetric center,
or an acid which is optically active with respect to axial asymmetry, and represented by the formula (XIIc):

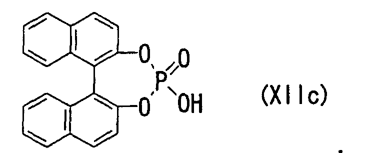

12. A compound represented by the formula (IX'): 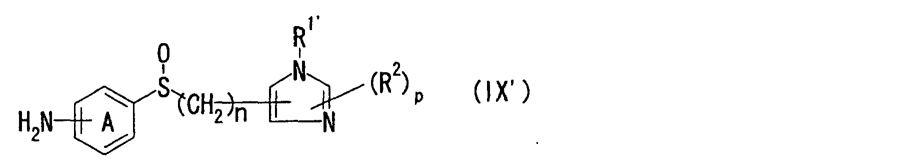 wherein R^{1'} represents an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3; and
p represents an integer of 0 to 2,
or a salt thereof.

13. A process for preparing a compound represented by the formula (IX): 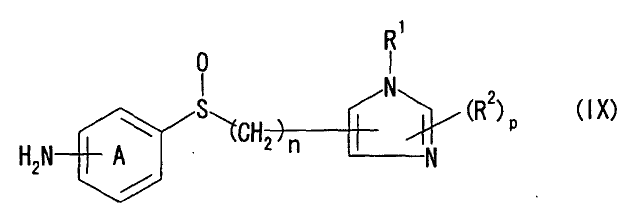 wherein R¹ represents a hydrogen atom, an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3; and
p represents an integer of 0 to 2,
or a salt thereof, which comprises subjecting a compound represented by the formula (VIII): 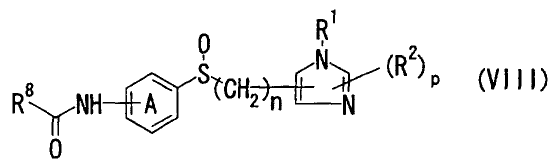 wherein R⁸ represents a hydrogen atom, an optionally substituted lower alkyl group, an optionally substituted aryl group, an optionally substituted aralkyl group or -OR¹⁰ wherein R¹⁰ represents an optionally substituted lower alkyl group, an optionally substituted aryl group or an optionally substituted aralkyl group; and
the other symbols are as defined above,
or a salt thereof, to a deprotection reaction.

14. A process for preparing a compound represented by the formula (IX): 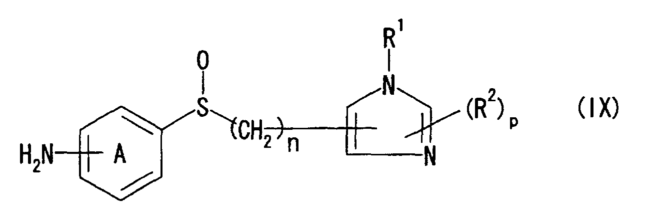 wherein R¹ represents a hydrogen atom, an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3; and
p represents an integer of 0 to 2,
or a salt thereof, wherein a compound represented by the formula (X): 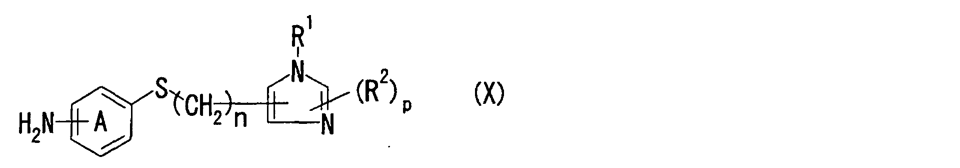 wherein each symbol is as defined above,
or a salt thereof is oxidized.

15. A compound represented by the formula (VIII): 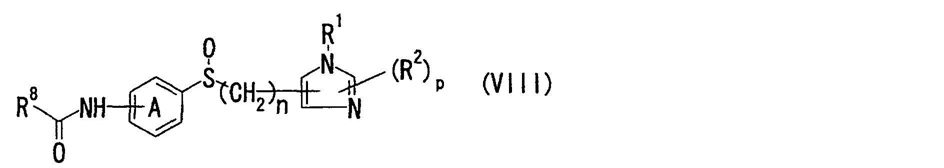 wherein R¹ represents a hydrogen atom, an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
R⁸ represents a hydrogen atom, an optionally substituted lower alkyl group, an optionally substituted aryl group, an optionally substituted aralkyl group or -OR¹⁰ wherein R¹⁰ represents an optionally substituted lower alkyl group, an optionally substituted aryl group or an optionally substituted aralkyl group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3; and
p represents an integer of 0 to 2,
or a salt thereof.

16. A process for preparing a compound represented by the formula (VIII): 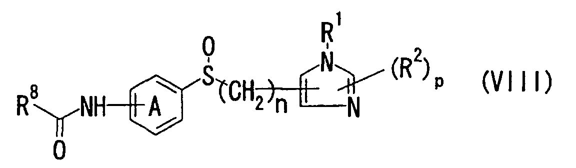 wherein R¹ represents a hydrogen atom, an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
R⁸ represents a hydrogen atom, an optionally substituted lower alkyl group, an optionally substituted aryl group, an optionally substituted aralkyl group or -OR¹⁰ wherein R¹⁰ represents an optionally substituted lower alkyl group, an optionally substituted aryl group or an optionally substituted aralkyl group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3; and
p represents an integer of 0 to 2,
or a salt thereof, which comprises oxidizing a compound represented by the formula (VII): 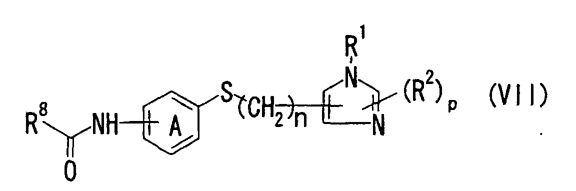 wherein each symbol is as defined above,
or a salt thereof.

17. A compound represented by the formula (VII'): 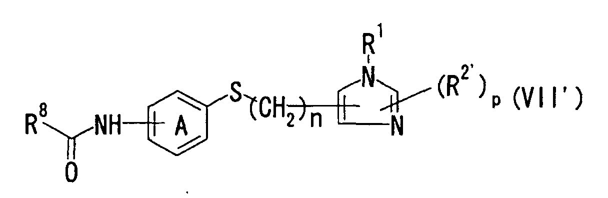 wherein R¹ represents a hydrogen atom, an optionally substituted aliphatic hydrocarbon group or an optionally substituted aromatic group;
R^{2'} represents a halogen atom, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
R⁸ represents a hydrogen atom, an optionally substituted lower alkyl group, an optionally substituted aryl group, an optionally substituted aralkyl group or -OR¹⁰ wherein R¹⁰ represents an optionally substituted lower alkyl group, an optionally substituted aryl group or an optionally substituted aralkyl group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3; and
p represents an integer of 0 to 2,
or a salt thereof.

18. A process for preparing a compound represented by the formula (VII): 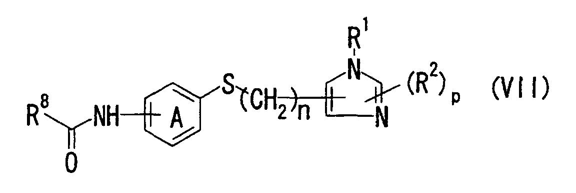 wherein R¹ represents a hydrogen atom, an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
R⁸ represents a hydrogen atom, an optionally substituted lower alkyl group, an optionally substituted aryl group, an optionally substituted aralkyl group or -OR¹⁰ wherein R¹⁰ represents an optionally substituted lower alkyl group, an optionally substituted aryl group or an optionally substituted aralkyl group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3; and
p represents an integer of 0 to 2,
or a salt thereof, which comprises reacting a compound represented by the formula (V): 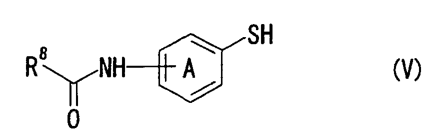 wherein each symbol is as defined above,
or a salt thereof, with a compound represented by the formula (VI): 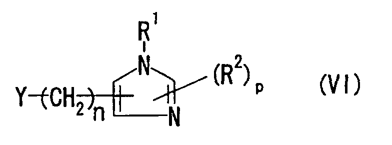 wherein Y represents a halogen atom or a group represented by the formula -OSO₂-R⁹ wherein R⁹ represents a lower alkyl group or an optionally substituted aryl group; and
the other symbols are as defined above,
or a salt thereof.

19. A compound represented by the formula (X'): 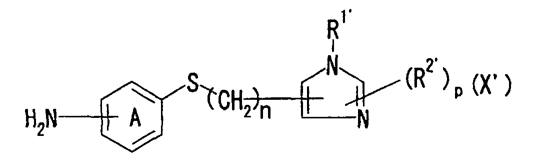 wherein R^{1'} represents an optionally substituted aliphatic hydrocarbon group or an optionally substituted aromatic group;
R^{2'} represents a halogen atom, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3; and
p represents an integer of 0 to 2,
or a salt thereof.

20. A process for preparing a compound represented by the formula (X): 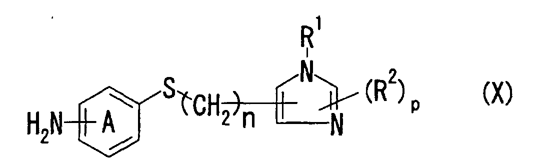 wherein R¹ represents a hydrogen atom, an optionally substituted aliphatic hydrocarbon group or an optionally substituted aromatic group;
R² represents a halogen atom, a nitro group, a cyano group, an optionally substituted alkyl group, an optionally substituted cycloalkyl group, an optionally substituted hydroxyl group, an optionally substituted thiol group (the sulfur atom may be oxidized to form a sulfinyl group that may be substituted or a sulfonyl group that may be substituted), an optionally substituted amino group, an optionally substituted acyl group, an optionally esterified carboxyl group or an optionally substituted aromatic group;
the ring A represents a benzene ring which may be substituted with a halogen atom, a C₁₋₄ alkyl group which may be substituted with a halogen atom or a C₁₋₄ alkoxy group which may be substituted with a halogen atom;
n represents an integer of 0 to 3; and
p represents an integer of 0 to 2,
or a salt thereof, which comprises reacting a compound represented by the formula (IV): 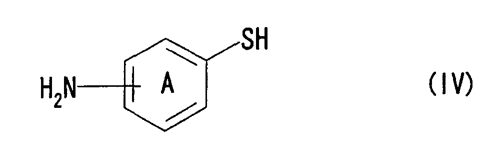 wherein the ring A is as defined above,
or a salt thereof, with a compound represented by the formula (VI): 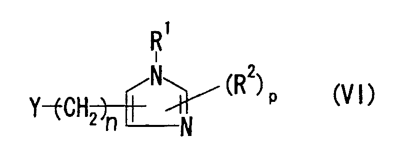 wherein Y represents a halogen atom or a group represented by the formula -OSO₂-R⁹ wherein R⁹ represents a lower alkyl group or an optionally substituted aryl group; and
the other symbols are as defined above,
or a salt thereof.
